(19) Europäisches Patentamt
European Patent Office
Office européen des brevets

(11) Publication number: **0 418 071 B1**

# (12) EUROPEAN PATENT SPECIFICATION

(45) Date of publication of patent specification: **26.04.95**

(51) Int. Cl.$^6$: **C07C 323/60**, C07D 239/58, C07D 213/75, C07D 213/70, C07C 323/52, A61K 31/16, A61K 31/33, C07D 307/64, C07D 277/74, C07D 277/36, C07D 215/36

(21) Application number: **90310009.7**

(22) Date of filing: **13.09.90**

Divisional application 94200437.5 filed on 13/09/90.

The file contains technical information submitted after the application was filed and not included in this specification

## (54) New N-aryl and N-heteroarylamide and urea derivatives as inhibitors of acyl coenzyme A: cholesterol acyl transferase.

(30) Priority: **15.09.89 PCT/US89/04033**

(43) Date of publication of application:
**20.03.91 Bulletin 91/12**

(45) Publication of the grant of the patent:
**26.04.95 Bulletin 95/17**

(84) Designated Contracting States:
**AT BE CH DE DK ES FR GB GR IT LI LU NL SE**

(56) References cited:
**EP-A- 0 283 742**
**WO-A-91/04027**

(73) Proprietor: **PFIZER INC.**
**235 East 42nd Street**
**New York, N.Y. 10017 (US)**

(72) Inventor: **McCarthy, Peter A.**
**235 North Anguilla**
**RR5, Pawcatuck,**
**Connecticut (US)**
Inventor: **Walker, Frederick J.**
**5, Maynard Hill Road**
**Preston,**
**Connecticut (US)**
Inventor: **Truong, Thien**
**14, Cinnamon Ridge**
**Old Saybrook,**
**Connecticut 06475 (US)**
Inventor: **Hamanaka, Ernest S.**
**40 Eagle Ridge Drive**
**Gales Ferry,**
**Connecticut (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 418 071 B1

**CHEMICAL ABSTRACTS, vol. 95, 1981, page 290, abstract no. 146539r, Columbus, Ohio, US; H. WADA et al.: "Gas chromatographic and mass spectrometric analysis of molecular species of bacterionema mycolic acids from Bacterionema matruchotii", & FEMS MICROBIOL. LETT. 1981, 11(2-3), 187-92**

Inventor: **Chang, George**
**One Winthorp Hill Road**
**Ivoryton,**
**Connecticut 06442 (US)**

(74) Representative: **Moore, James William, Dr.**
**Pfizer Limited**
**Ramsgate Road**
**Sandwich**
**Kent CT13 9NJ (GB)**

## Description

The present invention relates to new N-heteroarylamide derivatives, pharmaceutical compositions comprising such compounds, and the use of such compounds to inhibit intestinal absorption of cholesterol, lower serum cholesterol and reverse the development of atherosclerosis. The compounds are inhibitors of acyl coenzyme A: cholesterol acyltransferase (ACAT).

Cholesterol that is consumed in the diet (dietary cholesterol) is absorbed as free cholesterol by the mucosal cells of the small intestine. It is then esterified by the enzyme ACAT, packaged into particles known as chylomicrons, and released into the bloodstream. Chylomicrons are particles into which dietary cholesterol is packaged and transported in the bloodstream. By inhibiting the action of ACAT, the compounds of this invention prevent intestinal absorption of dietary cholesterol and thus lower serum cholesterol levels. They are therefore useful in preventing atherosclerosis, heart attacks and strokes.

By inhibiting the action of ACAT, the compounds of the present invention also enable cholesterol to be removed from the walls of blood vessels. This activity renders such compounds useful in slowing or reversing the development of atherosclerosis as well as in preventing heart attacks and strokes.

Other inhibitors of ACAT are referred to in United States Patents 4,716,175 and 4,743,605 (a divisional of the '175 patent) and in the European Patent Applications having publication numbers 0 242 610, 0 245 687 and 0 252 524. Certain ureas and thioureas as antiatherosclerosis agents are referred to in United States Patent 4,623,662.

The present invention relates to compounds of the formula

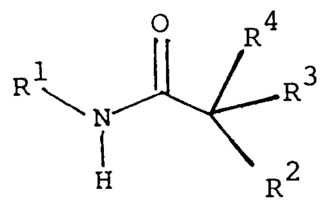

I

wherein $R^1$ is:

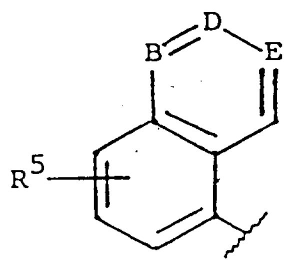

XXIV

$R^2$, $R^3$ and $R^4$ may be the same or different, and

(a) are selected from the group consisting of hydrogen, $(C_1-C_4)$ alkyl, A, $XR^{10}$, phenyl-$(C_1-C_7)$ alkyl, and $(C_5-C_6)$ cycloalkyl-$(C_1-C_6)$ alkyl, with the proviso that at least one of $R^2$, $R^3$ and $R^4$ must be A; or

(b) $R^2$ and $R^3$ together with the carbon to which they are attached form a cyclic or bicyclic system selected from the group consisting of $(C_3-C_7)$ cycloalkyl, $(C_3-C_7)$ cycloalkenyl, $(C_6-C_{14})$ bicycloalkyl, $(C_6-C_{14})$ bicycloalkenyl, and aryl-fused and heteroaryl-fused systems containing 8 to 15 carbon atoms, one ring of any of said aryl-fused and heteroaryl-fused systems being aromatic and the ring containing the carbon to which $R^2$ and $R^3$ are attached being non-aromatic, one of the carbons of said aromatic ring being optionally replaced by sulfur or oxygen, one or more carbons of said non-aromatic ring being optionally replaced by sulfur or oxygen, and one or more carbons of said aromatic ring being optionally replaced by nitrogen; one or two carbons of said cycloalkyl or bicycloalkyl groups being optionally replaced by sulfur or oxygen, and said cyclic or bicyclic system being optionally substituted with one to five substituents independently selected from the group consisting of phenyl, substituted phenyl, $(C_1-C_6)$

alkyl and A, with the proviso that one and only one of said substituents is A, and one and only one of said substituents is phenyl or substituted phenyl, said substituted phenyl being substituted with one or more substituents independently selected from the group consisting of ($C_1$-$C_6$) alkyl, ($C_1$-$C_6$) alkylthio, halogen and trifluoromethyl; and $R^4$ is hydrogen, $XR^{10}$ or A;

A is a hydrocarbon containing 4 to 16 carbons and 0, 1 or 2 double bonds:

X is O, S, SO, $SO_2$, NH, $NR^{23}CO$ or $NSO_2R^{24}$, wherein $R^{23}$ is hydrogen or ($C_1$-$C_6$) alkyl and $R^{24}$ is ($C_1$-$C_6$) alkyl, phenyl or ($C_1$-$C_3$) alkyl-phenyl;

$R^5$ is ($C_1$-$C_6$) alkylthio, which may be attached to either ring of the bicyclic ring system.

$R^{10}$ is selected from the group consisting of ($C_4$-$C_{12}$) cycloalkyl, ($C_4$-$C_{12}$) straight or branched alkyl, ($C_4$-$C_{12}$) cycloalkyl-($C_1$-$C_6$) alkyl, phenyl- ($C_1$-$C_6$) alkyl, (substituted phenyl)-($C_1$-$C_6$) alkyl, ($C_1$-$C_6$) alkyl-phenyl, ($C_1$-$C_6$) alkyl-(substituted phenyl), optionally substituted thiazoles, optionally substituted benzothiazoles, and optionally substituted pyridines; wherein the substituents on the substituted phenyl, substituted thiazoles, substituted benzothiazoles and substituted pyridines are selected from the group consisting of ($C_1$-$C_4$) alkoxy, ($C_1$-$C_4$) alkylthio, ($C_1$-$C_6$) alkyl, halo and trifluoromethyl;

B, D and E are selected from the group consisting of nitrogen and carbon, with the proviso that one or two of B, D and E is nitrogen.

Examples of said aryl-fused and heteroaryl-fused systems are:

1,2,3,4-tetrahydronapthalene,
5,6,7,8,9-pentahydrobenzocycloheptene,
5,6,7,8,9,10-hexahydrobenzocyclooctene,
4,5,6-trihydro-1-thiapentalene,
4,5,6-trihydro-2-thiapentalene,
4,5,6,7-tetrahydrobenzo[b]thiophene,
4,5,6,7-tetrahydrobenzo[c]thiophene,
4,5,6-trihydro-1-oxapentalene,
4,5,6,7-tetrahydrobenzo[b]furan,
4,5,6-trihydro-1-azapentalene,
4,5,6,7-tetrahydrobenzo[b]pyrrole,
4,5,6-trihydro-1-oxa-3-azapentalene,
4,5,6,7-tetrahydrobenzo[d]oxazole,
4,5,6-trihydro-1-thia-3-azapentalene
4,5,6,7-tetrahydrobenzo[d]thiazole,
4,5,6-trihydro-1-oxa-2-azapentalene,
4,5,6,7-tetrahydrobenzo[d]oxazole,
4,5,6-trihydro-1-thia-2-azapentalene,
4,5,6,7-tetrahydrobenzo[d]thiazole,
4,5,6-trihydro-1,2-diazapentalene,
4,5,6,7-tetrahydrobenzo[d]pyrazole,
4,6-diazaindane and
5,6,7,8-tetrahydroquinazoline.

Unless otherwise indicated, the term "halo", as used herein, includes fluoro, chloro, bromo and iodo.

Unless otherwise indicated, the term "alkyl", as used herein, may be straight, branched or cyclic, and may include straight and cyclic moieties as well as branched and cyclic moieties.

Unless otherwise indicated, the term "one or more substituents", as used herein, refers to from one to the maximum number of substituents possible based on the number of available bonding sites.

The term "one or more carbons of said non-aromatic ring", as used herein, refers to from one to all of the carbon atoms that are part of the non-aromatic ring of any of the aryl-fused or heteroaryl-fused systems described above, and not part of the aromatic ring of said aryl-fused system.

The term "one or more carbons of said aromatic ring", as used herein, refers to from one to all of the carbon atoms that are part of the aromatic ring of any of the aryl-fused and heteroaryl-fused systems described above, or are part of both said aromatic and non-aromatic rings of said aryl-fused and heteroaryl-fused system.

The compounds of formula I may have optical centers and therefore may occur in different stereoisomeric configurations. The invention includes all stereoisomers of such compounds of formula I, including mixtures thereof.

Preferred compounds of formula I are those wherein $R^1$ is 6-methylthioquinolin-5-yl, or 6-methylthioisoquinolin-5-yl.

Other preferred compounds of formula I are those wherein:

$R^2$ is hexylthio, $R^3$ is octyl and $R^4$ is hydrogen; or

$R^2$ and $R^3$ together with the carbon to which they are attached form an indan-2-yl ring, and $R^4$ is 2-decyl; or

$R^2$ and $R^3$ together with the carbon to which they are attached form a 1,2,3,4-tetrahydronaphth-2-yl ring and $R^4$ is nonyl.

Specific preferred compounds of formula I are:

N-(6-methylthioquinolin-5-yl)-2-(hexylthio) decanoic amide;

N-(6-methylthioisoquinolin-5-yl)-2-(hexylthio) decanoic amide;

N-(6-methylthioquinolin-5-yl)-2-(4-(3-methylpropyl)phenoxy) nonanoic amide;

(2S)-N-[6-(methylthio)quinolin-5-yl]-2-hexylthiodecanoic amide, and

N-(6-methylthioquinolin-5-yl)-2-octyl-1,3-dithian-2-yl carboxamide .

Other compounds of formula I are:

N-(6-isopropylthioquinolin-5-yl)-2-(4-ethylthiophenoxy)nonanoicamide;

N-(6-methylthioquinolin-5-yl)-2-hexyloxydecanoic amide;

N-(6-methylthioquinolin-5-yl)-2-heptylnonanoic amide;

The present invention also relates to a pharmaceutical composition for inhibiting ACAT, inhibiting intestinal absorption of cholesterol, reversing or slowing the development of atherosclerosis, or lowering the concentration of serum cholesterol in a mammal, including a human, comprising an amount of a compound of the formula I, or a pharmaceutically acceptable salt thereof, effective in inhibiting ACAT, inhibiting intestinal absorption of cholesterol, reversing or slowing the development of atherosclerosis, or lowering the concentration of serum cholesterol, and a pharmaceutically acceptable carrier.

Examples of pharmaceutically acceptable acid addition salts of the compounds of formula I salts are the salts of hydrochloric acid, p-toluenesulfonic acid, fumaric acid, citric acid, succinic acid, salicyclic acid, oxalic acid, hydrobromic acid, phosphoric acid, methanesulfonic acid, tartaric acid, di-p-toluoyl tartaric acid, and mandelic acid.

Reaction schemes 1-4 below illustrate the synthesis of the compounds of this invention.

Scheme 5 below illustrates the synthesis of certain 5-aminoquinolines and 5-aminoisoquinolines used as reactants in scheme 1-4.

Except where otherwise stated, $R^1$, $R^2$, $R^3$, $R^4$, $R^5$, $R^{10}$, X, A, B and D in the reaction schemes and discussion that follows are defined as above and p is O, 1 or 2.

SCHEME 1

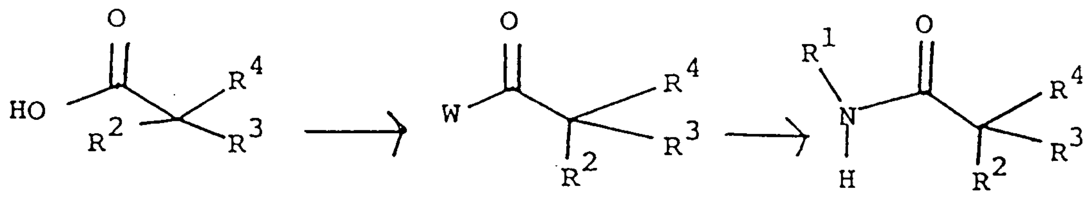

III IV I

SCHEME 2

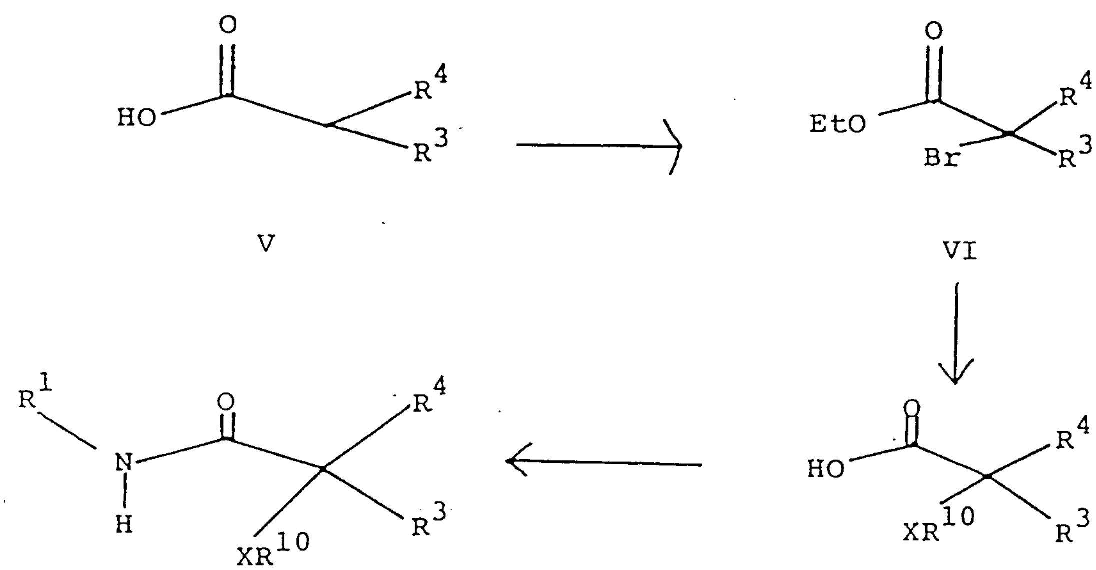

V VI

IB VII

SCHEME 3

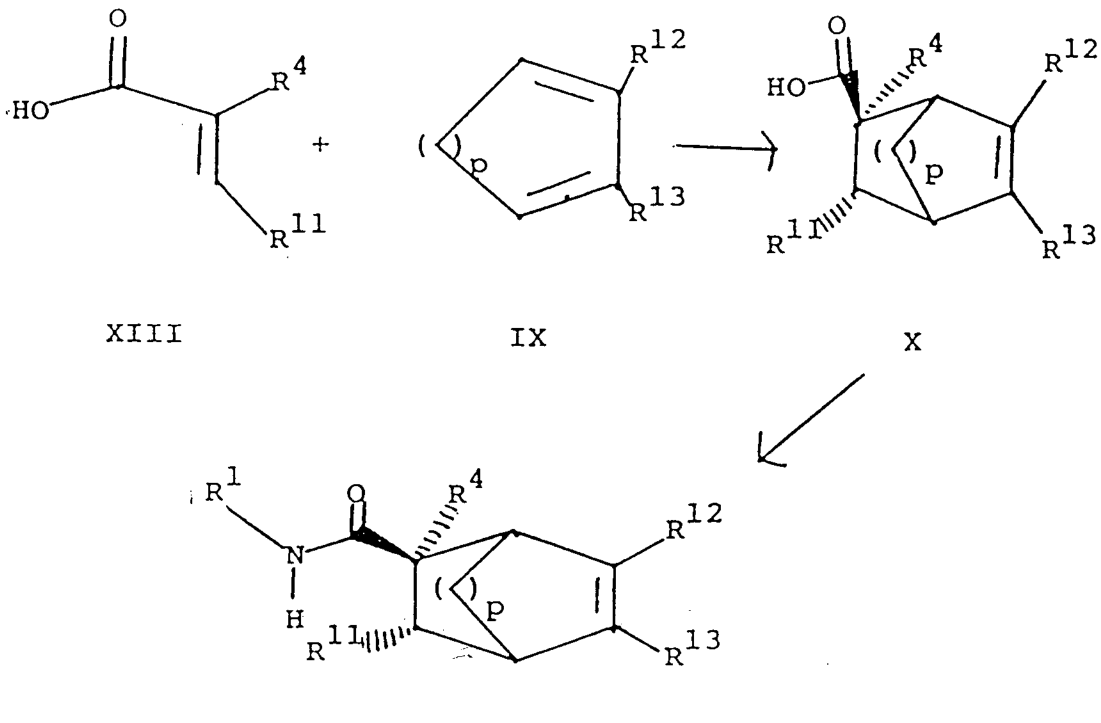

XIII IX X

IC

SCHEME 4

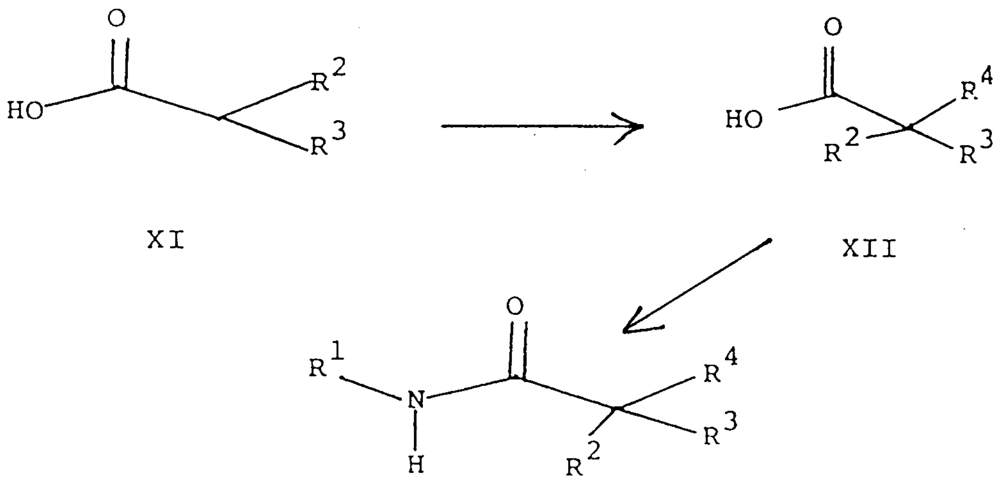

XI XII

I

SCHEME 5

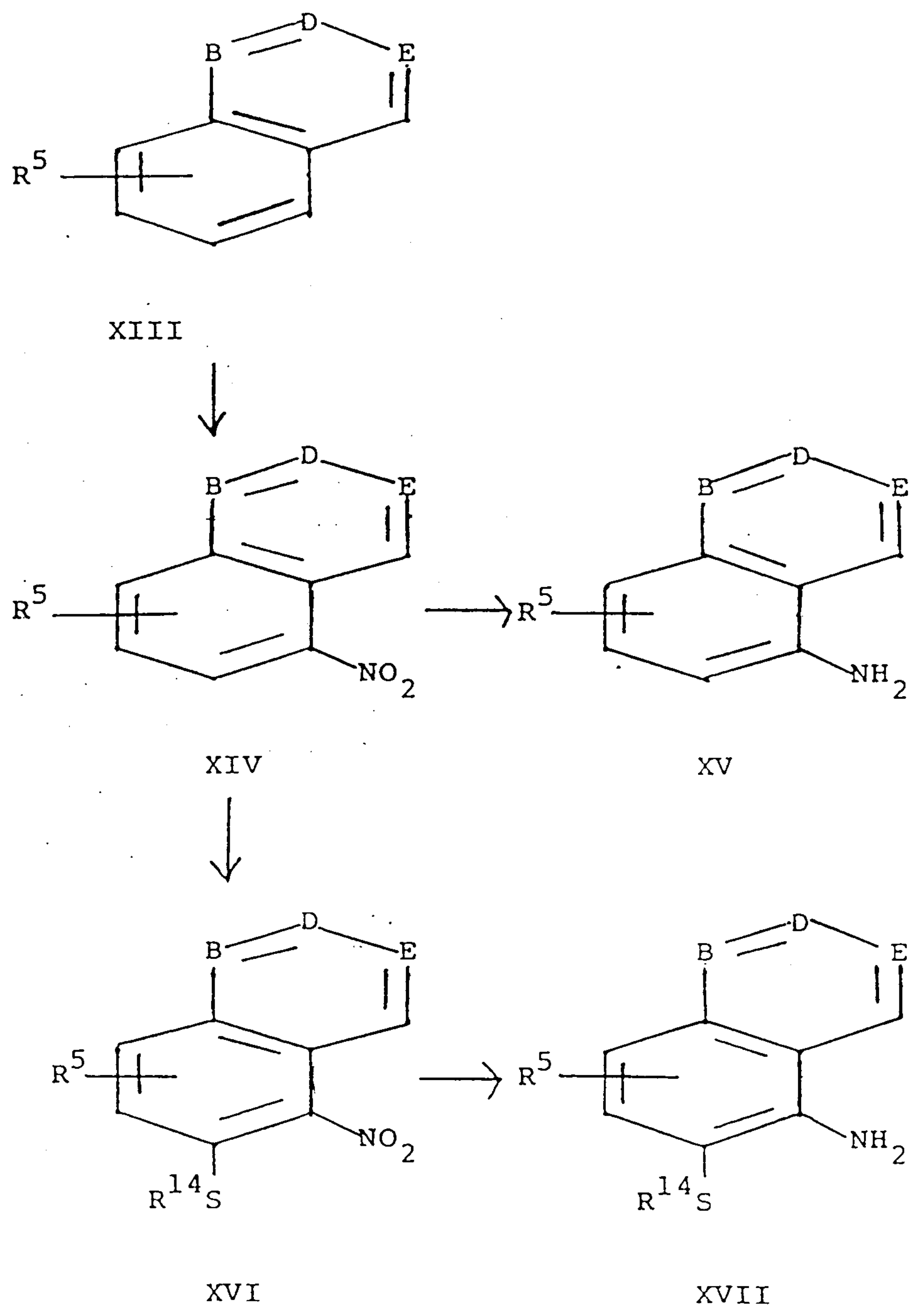

XIII

XIV

XV

XVI

XVII

Scheme 1 represents the synthesis of amides of the present invention having the formula I from the corresponding carboxylic acid having the formula III. An acid of formula III is first converted to the corresponding acid halide of formula IV, wherein W is chloro or bromo, by reacting it with a chlorinating or brominating agent. Examples of suitable chlorinating and brominating agents are oxalyl chloride, oxalyl bromide, thionyl chloride, thionyl bromide, phosphorous trichloride, phosphorous tribromide, phosphorous pentachloride, phosphorous pentabromide, phosphorous oxychloride, and phosphorous oxybromide. This reaction is typically carried out in the absence of a solvent or, alternatively, in the presence of a halogenated hydrocarbon solvent such as methylene chloride, for from about .5 to 48 hours (preferably from about 2 to 18 hours) at a temperature from about 0-250 °C (preferably at the reflux temperature of the reaction mixture). The acid halide so formed is then converted to the corresponding amide of the formula IA

by reacting it with an amine of the formula $R^1NH_2$ and an acid scavenger such as dimethylaminopyridine, pyridine or triethylamine. This reaction is typically carried out in the absence of a solvent or in the presence of an inert solvent such as tetrahydrofuran or methylene chloride for from about 0.25 to 144 hours (preferably from about 2 to 72 hours) at a temperature from about -78 to 350 °C (preferably from about -20 to the reflux temperature of the reaction mixture).

Compounds of the formula I, wherein $R^2$ is $XR^{10}$, one of $R^3$ and $R^4$ is hydrogen and the other is selected from hydrogen, $(C_1\text{-}C_4)$ alkyl or A, i.e., compounds of the formula IB, may be prepared as illustrated in scheme 2. Referring to scheme 2, a carboxylic acid of the formula V, wherein one of $R^3$ and $R^4$ is hydrogen and the other is selected from hydrogen, $(C_1\text{-}C_4)$ alkyl or A, is reacted for about 3 hours with thionyl chloride using no solvent, at the reflux temperature of the reaction mixture. Bromine and a catalytic amount of iodine are then added to the reaction mixture, and the resulting mixture is brought to reflux. After refluxing for about 18 hours, ethanol is added and the mixture is refluxed for about 1 more hour to produce a bromoester of the formula VI, wherein $R^3$ and $R^4$ are defined as they are for formula V above. The bromoester of formula VI is then converted to an ester having the same formula as formula VI except that the substituent -Br is replaced by the substituent $-XR^{10}$, (hereinafter referred to as formula VI'), by reacting it with a compound of the formula $HXR^{10}$ and a base such as potassium carbonate or sodium hydride in an aprotic, polar solvent such as dimethylformamide, acetone or tetrahydrofuran, for about .5 to 48 hours (preferably from about 4 to 18 hours) at a temperature from about -78 to 350 °C (preferably from about 0 °C to the reflux temperature of the reaction mixture). An acid of the formula VII, wherein $R^3$ and $R^4$ are defined as they are for formulas V and VI above, is then prepared by reacting the ester having formula VI' with a hydroxide such as sodium hydroxide. This reaction is typically carried out overnight in an lower alcohol solvent such as methanol or ethanol, at a temperature from about -78 to 350 °C (preferably from about 20 °C to the reflux temperature of the reaction mixture).

The acid of formula VII so prepared is then converted to an amide of the formula IB, wherein $R^3$ and $R^4$ are defined as they are for formulae V, VI and VII above, by the acid to amide synthesis illustrated in scheme 1 and described above.

Compounds of the formula IB, may be prepared, alternatively, by the following method. A compound of the formula V, as illustrated in scheme 2 and defined above, is reacted with thionyl chloride followed by bromine and a catalytic amount of iodine as described above, but quenching the reaction with water instead of ethanol, to form a compound of the formula $HOOCCBrR^3R^4$, wherein $R^3$ and $R^4$ are defined as they are for formula V. This compound is then converted, sequentially, to the corresponding acid chloride of the formula $ClCOCBrR^3R^4$ and the corresponding amide of the formula $R^1NHCOCBrR^3R^4$, wherein $R^3$ and $R^4$ are defined as they are for formula V, by the acid to amide synthesis illustrated in scheme 1 and described above. The amide of the formula $R^1NHCOCBrR^3R^4$ so formed is then reacted with a compound of the formula $HXR^{10}$ and a base such as potassium carbonate and sodium hydride to form a compound having the formula IB, wherein $R^3$ and $R^4$ are defined as they are for formula V. This reaction is typically carried out in an aprotic, polar solvent such as dimethylformamide, acetone or tetrahydrofuran, for from about 0.5 to 48 hours (preferably from about 4 to 18 hours). The reaction may be carried out at temperatures ranging from about -78 to 350 °C (preferably from about 0 °C to the reflux temperature of the reaction mixture).

Scheme 3 illustrates the preparation of compounds of formula I, wherein $R^4$ is hydrogen or A, and $R^2$ and $R^3$, together with the carbon to which they are attached, form the bicyclic ring system

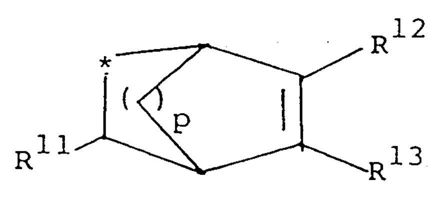

wherein the asterisk designates the carbon to which $R^2$ and $R^3$ are attached, and each of $R^{12}$ and $R^{13}$ are independently selected from the group consisting of hydrogen and $(C_1\text{-}C_4)$ alkyl, or $R^{12}$ and $R^{13}$, together with the carbons to which they are attached, form a benzene ring.

As illustrated in scheme 3, a Diels-Alder reaction is carried out between an acid of the formula XIII, wherein $R^{11}$ is A, hydrogen, phenyl or substituted phenyl, and wherein $R^{11}$ and the carboxyl group are trans to each other, and a diene of the formula IX, wherein $R^{12}$ and $R^{13}$ are as defined above. This reaction is typically carried out in a hydrocarbon solvent such as toluene, using a catalytic amount of an antioxidant such as hydroquinone. The reagents are generally reacted for about 1 to 10 days (preferably for about 3 to 5 days) in a sealed, high pressure apparatus at a temperature from about room temperature to 350 °C

(preferably from about 100 to 150 ° C). The reaction yields an acid of the formula X, which can be converted to the corresponding amide of the formula IC, wherein the carbons to which $R^{12}$ and $R^{13}$ are attached are bonded by a carbon-carbon double bond, by the acid to amide synthesis illustrated in scheme 1 and described above. The amide of formula IC so formed can be converted to an amide of the formula IC, wherein the carbons to which $R^{12}$ and $R^{13}$ are attached are bonded by a carbon-carbon single bond, by reacting it with a reducing agent such as hydrogen. Typically, the reduction is carried out using hydrogen gas in a high pressure apparatus, in an inert solvent such as acetic acid, and in the presence of a hydrogenation catalyst such as palladium on carbon.

The reduction maybe carried out at temperatures ranging from about -20 to 250 ° C (preferably at room temperature). Fifty p.s.i. (3.45 bar) of hydrogen is the preferred pressure, through pressures greater than or equal to 1 atmosphere are suitable. The corresponding compound of formula IC, wherein the carboxyl group and $R^{11}$ are cis to each other, may be prepared in a similar manner, but using the corresponding cis isomer of the acid of formula XIII.

When the procedure of scheme 3 described above is used to prepare a compound of formula IC wherein $R^{12}$ and $R^{13}$, together with the carbon to which they are attached, form a benzene ring, the diene of formula IX is generally generated in situ in the presence of the acid of formula XIII or its ester by heating a mixture of 1,3-dihydrobenzo[c]thiophene 2,2-dioxide and such acid or ester. This reaction is typically carried out at a temperature of from about 235 to 300 ° C (preferably from about 250 to 265 ° C) under nitrogen for approximately from 0.5 to 24 hours (preferably for about 2 hours).

Scheme 4 illustrates the preparation of compounds of the formula I, wherein $R^4$ is ($C_1$-$C_4$) alkyl or A, and $R^2$ and $R^3$ are each independently selected from the group consisting of hydrogen, ($C_1$-$C_4$) alkyl, A or $XR^{10}$; or $R^2$ and $R^3$, together with the carbon to which they are attached, form a cyclic or bicyclic system selected from the group consisting of ($C_3$-$C_7$) cycloalkyl, ($C_6$-$C_{14}$) bicycloalkyl, one or two carbons of said cycloalkyl and bicycloalkyl groups being optionally replaced with oxygen or sulfur; and aryl-fused and heteroaryl-fused systems containing 8 to 15 carbon atoms, one ring of any of said aryl-fused and heteroaryl-fused systems being aromatic and the ring containing the carbon to which $R^2$ and $R^3$ are attached being non-aromatic, one of the carbons of said aromatic ring being optionally replaced by sulfur or oxygen, one or more carbons of said non-aromatic ring being optionally replaced by sulfur or oxygen, and one or more carbons of said aromatic ring being optionally replaced by nitrogen. It also illustrates the preparation of compounds of the formula I, wherein

$R^4$ is $XR^{10}$, and $R^2$ and $R^3$, together with the carbon to which they are attached, form a cyclic or bicyclic system as defined immediately above. All compounds of the invention illustrated in scheme 4 are prepared by the following procedure.

An acid of the formula XI, wherein $R^2$ and $R^3$ are each independently selected from the group consisting of hydrogen, ($C_1$-$C_4$) alkyl, A or $XR^{10}$, or $R^2$ and $R^3$ together with the carbon to which they are attached, form a cyclic or bicyclic system as defined immediately above; is reacted with a base such as lithium diisopropylamide or hexamethyldisilazide, with or without an additive such as hexamethylphosphorous triamide, in a dry inert solvent such as tetrahydrofuran, and then reacted with a compound of the formula $R^4$Hal, wherein Hal is halogen and $R^4$ is ($C_1$-$C_7$) alkyl or A. The reaction is typically carried out at a temperature from about -78 to 40 ° C (preferably from about -78 ° C to room temperature) for about 0.5 to 48 hours (preferably for about 1.5 to 17 hours: 0.5 to 1 hour to generate the dianion of formula XI and 1 to 16 hours for the alkylation). The product of the reaction is an acid of the formula XII, wherein $R^2$ and $R^3$ are as defined immediately above, and $R^4$ is ($C_1$-$C_7$) alkyl or A. The acid of formula XII so formed may be converted to the corresponding amide of tee formula ID, wherein $R^2$ and $R^3$ are as defined immediately above and $R^4$ is ($C_1$-$C_7$) alkyl or A, by the acid to amide synthesis illustrated in scheme 1 and described above.

Compounds of the formula I , wherein $R^4$ is $XR^{10}$, are prepared by the same procedure as that described above for the preparation of compounds of the formula I wherein $R^4$ is ($C_1$-$C_7$) alkyl or A, with one modification. The dianion of formula XI is reacted with a compound of formula $R^{10}SSR^{10}$ instead of $HalR^4$. This reaction produces an acid of the formula XII, wherein $R^4$ is $XR^{10}$. The acid of the formula XII so formed can then be converted to the corresponding amide of formula I by the acid to amide synthesis illustrated in scheme 1 and described above.

The preparation of certain 5-aminoquinolines and 5-aminoisoquinolines used as reactants in scheme 1 is illustrated in scheme 5. Referring to scheme 5, 5-aminoquinolines and isoquinolines of the formulae XV and XVII may be prepared as follows. A quinoline or isoquinoline of the formula XIII is nitrated at the 5 position, respectively, by reacting it with a nitrating agent such as nitric acid or potassium nitrate with or without an acid catalyst such as sulfuric acid, for from about 2 to 16 hours at a temperature from about 0-100 ° C. The nitro compound of formula XIV so formed is then reduced using a reducing agent such as

stannous chloride, iron, zinc, or hydrogen gas with an appropriate catalyst, with or without an acid catalyst such as hydrochloric acid, for from about 2 to 16 hours at a temperature from about 0-100 °C, to yield the corresponding 5-aminoquinoline or 5-amioisoquinoline of formula XV.

Compounds of the formula XVII, wherein $R^5$ is $-SR^{14}$ and is attached to the quinoline or isoquinoline ring at the 6 position, and wherein $R^{14}$ is $(C_1-C_6)$ alkyl, may be prepared as follows. A compound of the formula XIV, wherein $R^5$ is -Cl and is attached to the quinoline or the isoquinoline ring at the 6 position, is reacted with a compound of the formula $R^{14}SH$, wherein $R^{14}$ is as defined above, and a base such as sodium hydride, or such compound of the formula XIV is reacted with a compound of the formula $R^{14}SNa$, wherein $R^{14}$ is as defined above, in an inert solvent such as tetrahydrofuran, for about 4 to 16 hours at a temperature of from about -10 °C to room temperature. The preferred temperature is -10 °C. This reaction yields a compound of the formula XVI, which is then converted to the corresponding 5-aminoquinoline or isoquinoline of the formula XVII by the method described above for reduction of compounds of formula XIV.

Except where otherwise noted, pressure is not critical in any of the above reactions. Preferred temperatures for the above reactions were stated where known. In general, the preferred temperature for each reaction is the lowest temperature at which product will be formed. The preferred temperature for a particular reaction may be determined by monitoring the reaction using thin layer chromatography.

The novel compounds of formula I and the pharmaceutically acceptable salts thereof are useful as inhibitors of acyl coenzyme A: cholesterol acyltransferase (ACAT). As such they inhibit intestinal absorption of cholesterol in mammals and are useful in the treatment of high serum cholesterol in mammals, including humans. As used herein, treatment is meant to include both the prevention and alleviation of high serum cholesterol. The compound may be administered to a subject in need of treatment by a variety of conventional routes of administration, including orally, parenterally and topically. In general, these compounds will be administered orally or parenterally at dosages between about 0.5 and about 30 mg/kg body weight of the subject to be treated per day, preferably from about .08 to 5 mg/kg. For an adult human of approximately 70 kg of body weight, the usual dosage would, therefore, be about 3.5 to about 2000 mg per day. However, some variation in dosage will necessarily occur depending on the condition of the subject being treated and the activity of the compound being employed. The person responsible for administration will, in any event, determine the appropriate dose for the individual subject.

A compound of formula I or a pharmaceutically acceptable salt thereof may be administered alone or in combination with pharmaceutically acceptable carriers, in either single or multiple doses. Suitable pharmaceutical carriers include inert solid diluents or fillers, sterile aqueous solution and various organic solvents. The resulting pharmaceutical compositions are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of a compound of formula I or a pharmaceutically acceptable salt thereof in sesame or peanut oil, aqueous propylene glycol, or in sterile aqueous solution may be employed. Such aqueous solutions should be suitably buffered if necessary and the liquid diluent first rendered isotonic with sufficient saline or glucose. Such solutions are especially suitable for intravenous, intramuscular, subcutaneous and intraperitioneal administration. In this connection, the sterile aqueous media employed are all readily available by standard techniques known to those skilled in the art.

The activity of the compounds of the present invention as ACAT inhibitors may be determined by a number of standard biological or pharmacological tests. For example, the following procedure was used to determine the ACAT inhibiting activity of compounds of formula I. ACAT was assayed in microsomes isolated from chow fed Sprague-Dawley rats according to Bilheimer, J.T., Meth. Enzymol., 111, ps 286-293 (1985), with minor modifications. Microsomes from rat liver were prepared by differential centrifugation and washed with assay buffer prior to use. The assay mixture contained 25 ul of BSA (40 mg/ml), 30 ul of rat liver microsome solution (100 ug microsomal protein), 20 ul of assay buffer (0.1 M $K_2PO_4$, 1.0 mM reduced Glutathione, pH 7.4), 20 ug of cholesterol in 100 ul of a 0.6% Triton WR-1339 solution in assay buffer, and

5 ul of test compound dissolved in 100% DMSO (total volume = 180 ul). The assay mixture was incubated for 30 min at 37°C. The reaction was started by the addition of 20 ul of 14C-Oleoyl-CoA (1000 uM, 2,000 dpm/nmol) and run for 15 min at 37°C. The reaction was stopped by the addition of 1 ml ETOH. The lipids were extracted into 4 ml hexane. A 3 ml aliquot was dried under $N_2$, and resuspended in 100 ul of chloroform. 50 ul of chloroform were spotted on a heat activated TLC plate and developed in hexane: diethyl ether: acetic acid (85:15:1, v:v:v). Incorporation of radioactivity into cholesteryl esters was quantified on a Berthold LB2842 Linear TLC Analyzer. ACAT inhibition was calculated relative to a DMSO control assay.

The activity of the compounds of formula I in inhibiting intestinal absorbtion of cholesterol may be determined by the procedure of Melchoir and Harvell, J. Lipid. Res., 26, 306-315 (1985).

The present invention is illustrated by the following examples. It will be understood, however, that the invention is not limited to the specific details of these examples. Melting points are uncorrected. Proton nuclear magnetic resonance spectra ($^1H$ NMR) and $C^{13}$ nuclear magnetic resonance spectra ($C^{13}$ NMR) were measured for solutions in deuterochoroform ($CDCl_3$) and peak positions are expressed in parts per million (ppm) downfield from tetramethylsilane (TMS). The peak shapes are denoted as follows: s, singlet; d, doublet; t, triplet; q, quartet; m, multiplet; br, broad; c, complex.

## EXAMPLE 1

### Ethyl 2-(4-n-Propylphenylthio)nonanoate

1.6 g (0.033 mole) sodium hydride (50% dispersion in mineral oil) was added to a solution of 5.0 g (0.033 mole) 4-propylthiophenol in 25 ml anhydrous dimethylformamide. After 15 minutes, 8.8 g (0.033 mole) ethyl 2-bromononanoate (prepared according to J. Labelled Compounds Radiopharm. 14, 713 (1978)) was added and the resulting mixture was stirred at room temperature overnight. The reaction mixture was then diluted with 150 ml ethyl acetate and the resulting mixture was washed with 5 x 60 ml water and then with 60 ml saturated aqueous sodium chloride solution. The ethyl acetate solution was dried over anhydrous sodium sulfate, filtered and concentrated in vacuo. The resulting oil was chromatographed on 600 g silica gel, eluting with 7:3 hexane/methylene chloride to yield 9.0 g (81% yield) of the desired product as an oil.

$^1H$ NMR($CDCl_3$): $\delta$ 0.88 (c,6H); 1.1-1.5 (c,total 12H) including 1.12 (t,3H); 1.54-1.93 (c,4H); 2.54 (t,2H); 3.56 (q,1H); 4.07 (q,2H); 7.1 (d,2H); 7.36 (d,2H).

## EXAMPLE 1A

### 2-Hexylthiodecanoic Acid

17.3 g (0.36 mol) sodium hydride (50% dispersion in mineral oil) was added portionwise with stirring (gas evolution) to a solution of 26.8 ml. (0.19 mol) hexanethiol in 500 ml. anhydrous dimethylformamide. The mixture was stirred at toom temperature for 30 min., then 45.2g (0.18 mol) 2-bromodecanoic acid was added dropwise with stirring, keeping the temperature of the reaction mixture below 45°C. The reaction mixture was stirred at room temperature under nitrogen overnight. The mixture was then diluted with 500 ml. water and the pH of the resulting mixture was adjusted to 1.5 with 6N aqueous hydrochloric acid solution. This mixture was extracted with 3 x 400 ml. ethyl acetate and the combined ethyl acetate extracts were washed with 5 x 700 ml. water and 1 x 500 ml. brine. The ethyl acetate solution was dried over anhydrous sodium sulfate, filtered and concentrated in vacuo. The resulting oil was chromatographed on 2 kg. silica gel, eluting with methylene chloride to yield 35g (67% yield) of the desired product as an oil.

## EXAMPLE 1B

### Resolution of 2-hexylthiodecanoic acid

2-Hexylthiodecanoyl chloride was prepared by the procedure of Example 4A. A solution of 2-hexylthiodecanoyl chloride (2.39 g., 7.8 mmol) in 20 ml methylene chloride was added slowly with stirring under nitrogen to a solution of (R)-(-)-2-phenylglycinol (1.08 g, 7.9 mmol) and 4-dimethylaminopyridine (0.96 g, 7.9 mmol) in 80 ml methylene chloride at 5°C. The reaction mixture was stirred at room temperature overnight. Methylene chloride (100 ml.) was then added and the resulting solution was washed sequentially with 100 ml 1N aqueous hydrochloric acid solution, 100 ml water, 100 ml saturated aqueous sodium bicarbonate solution and 100 ml brine. The organic phase was dried over anhydrous sodium sulfate and

concentrated in vacuo to a solid residue (3.1 g). The diastereomers were separated by column chromatography on 800g silica gel using 1:1 hexane-diethyl ether as eluant. The less polar diastereomer (1.09 g, $[\alpha]_D^{RT}$ = -9.85° ($CH_3OH$); mp 98-100°C) and 0.99 g of the more polar diastereomer ($[\ ]_D^{RT}$ = -9.46° ($CH_3OH$); mp 105-108°C) were obtained along with 0.36 g of a mixture of diastereomers (total yield 76%). A solution of the less polar diastereomer (900 mg, 2.2 mmol) in 42 ml 1,4-dioxane and 42 ml 6N aqueous sulfuric acid solution was heated at 105°C under nitrogen for 15 hours. The reaction mixture was cooled to room temperature, diluted with 80 ml water and the resulting mixture was extracted with 4 x 60 ml ethyl acetate. The combined ethyl acetate extracts were washed with 60 ml brine, dried over anhydrous sodium sulfate and concentrated in vacuo to yield (S)-(-)-2-hexylthiodecanoic acid as an oil (634 mg., 99.6% yield); $[\alpha]_D^{RT}$ = -59.5° ($CH_3OH$).

In a similar manner, hydrolysis of the more polar diastereomer yielded 98.4% of (R)-(+)-2-hexylthiodecanoic acid as an oil; $[\alpha]_D^{RT}$ = +54.0° ($CH_3OH$).

EXAMPLE 2

Ethyl 2-(4-t-Butylphenylthio)octanoate

A mixture of 5.0 g (0.02 mole) ethyl 2-bromooctanoate, 3.37 g (0.02 mole) p-t-butylthiophenol and 3.31 g (0.24 mole) potassium carbonate in 70 ml acetone was refluxed under nitrogen overnight. The reaction mixture was cooled to room temperature and filtered and the filtrate was concentrated in vacuo. The residue was chromatographed on 500 g silica gel, eluting with 6:4 methylene chloride/hexane to yield 3.8 g (57% yield) of the desired product as an oil.

$^1H$ NMR($CDCl_3$): δ 0.88 (c,3H); 1.1-1.52 (c,total 20H) including 1.14 (t,3H) and 1.3 (s); 1.66-2.11 (c,2H); 358 (q,1H); 4.1 (q,2H); 7.36 (m,4H).

EXAMPLE 3 (ILLUSTRATIVE EXAMPLE)

2-(4-n-Propylphenylthio)nonanoic acid

A solution containing 5.7 (0.017 mole) of the title compound of Example 1, 35 ml of 1N aqueous sodium hydroxide solution (0.035 mole) and 3 ml methanol was refluxed overnight. The resulting solution was cooled to room temperature, acidified to pH 1.5 with 2N aqueous hydrochloric acid and extracted with 3 x 50 ml ethyl acetate. The combined ethyl acetate extracts were washed with 50 ml water and 50 ml saturated aqueous sodium chloride solution, dried over anhydrous sodium sulfate and concentrated in vacuo to yield the title compound as an oil (5.0 g, 96% yield) which was used in the subsequent reaction without further purification.

$^1H$ NMR($CDCl_2$): δ 0.88 (c, 6H); 1.17-1.54 (c, 12H); 1.54-1.92 (c, 4H); 2.53 (t, 2H); 3.54 (t, 1H); 7.1 (d, 2H); 7,37 (d, 2H).

EXAMPLE 4 (ILLUSTRATIVE EXAMPLE)

2-(4-n-Propylphenylthio)-N-(2,4,6-trimethoxyphenyl)nonanamide

1.54 g (5 mmole) of the title compound of Example 3 in 20 ml of thionyl chloride was refluxed for 3 hours and then concentrated to dryness in vacuo. 523 mg (1.6 mmole) of the resulting acid chloride was dissolved in 20 ml methylene chloride and to the solution was added 292 mg (1.6 mmole) 2,4,6-trimethoxyaniline and 195 mg (1.6 mmole) 4-dimethylaminopyridine. The resulting solution was stirred at room temperature overnight and then concentrated in vacuo. The residue was partitioned between 60 ml ethyl acetate and 20 ml 1N aqueous hydrochloric acid solution. The ethyl acetate layer was washed with 50 ml water and 50 ml saturated aqueous sodium chloride solution, dried over anhydrous sodium sulfate and concentrated in vacuo. The crude product was chromatographed on 100 g silica gel, eluting with 1:1 hexane/ethyl acetate to yield 370 mg (49% yield) of the title compound as a whitish solid.

EXAMPLE 4A (ILLUSTRATIVE EXAMPLE)

N-[2-methyl-4,6-bis(methylthio) pyrimidin-5-yl]-2-hexylthiodecanoic amide

A solution of 6.49 g (22.5 mmol) 2-hexylthiodecanoic acid in 40 ml thionyl chloride and 100 ml benzene was refluxed under nitrogen for 2.5 hours and then concentrated to dryness in vacuo. The resulting acid chloride (6.88 g, 22.5 mmol) was dissolved in 15 ml. methylene chloride and the solution was added dropwise to a solution of 4.63 g (23 mmol) 5-amino-4,6-bis(methylthio)-2-methylpyrimidine in 140 ml methylene chloride. The resulting solution was refluxed under nitrogen overnight. The reaction solution was then cooled, diluted with 140 ml methylene chloride and washed with 2 x 125 ml 3N aqueous hydrochloric acid solution, 1 x 125 ml water, 1 x 125 ml saturated aqueous sodium bicarbonate solution and 1 x 125 ml brine. The methylene chloride solution was dried over anhydrous sodium sulfate, filtered and concentrated to dryness in vacuo. The solid residue was recrystallised from diethyl ether yielding 5.35 g of the title compound, m.p. 99-101 °C. The filtrate was concentrated in vacuo and the residue was chromatographed on 400 g silica gel eluting with 9:1 hexane/ethyl acetate. Recrystallization of the product obtained by chromatography from diethyl ether yielded another 2.32 g of the title compound, m.p. 99 °-101 °C (total yield 72.4%).

$^1H$ NMR ($CDCl_3$): δ 0.87 (c, 6H); 1.21-1.84 (c, 21 H), 2.02 (m, 1H); 2.50 (s, 6H); 2.76 (s, 3H), 2.74 (t, 2H); 3.45 (t, 1H), 8.08 (s, 1H);

IR ($CHCl_3$): 2923, 2852, 1681, 1511, 1468, 1431, 1405 $cm^{-1}$.

EXAMPLE 4B (ILLUSTRATIVE EXAMPLE)

N-[2,4-bis(methylthio)-6-methylpyridin-3-yl]-2-hexylthiodecanoic amide

A solution of 4.19 g (13.7 mmol) 2-hexylthiodecanoyl chloride, prepared according to Example 4A, in 15 ml methylene chloride was added dropwise with stirring under nitrogen to a solution of 2.75 g (13.7 mmol) 3-amino-2,4-bis(methylthio)-6-methylpyridine in 30 ml pyridine cooled to 5 °C. The reaction mixture was stirred at room temperature under nitrogen overnight. Methylene chloride (250 ml) was then added to the reaction mixture and the resulting solution was washed with 3 x 50 ml 3N aqueous hydrochloric acid solution, 2 x 50 ml water, 1 x 50 ml saturated aqueous sodium bicarbonate solution and 1 x 50 ml brine. The methylene chloride solution was dried over anhydrous sodium sulfate, filtered and concentrated to dryness in vacuo.

The solid residue (6.5 g) was recrystallized from petroleum ether to yield 4.7 g of the title compound, m.p. 75-76.5 °C (72.8% yield).

$^1H$ NMR ($CDCl_3$): δ 0.86 (c, 6H); 1.16-1.74 (c, 21H); 2.04 (m, 1H); 2.4 (s, 3H); 2.48 (s, 3H); 2.5 (s, 3H); 2.77 (t, 2H); 3.45 (t, 1H); 6.65 (s, 1H); 8.14 (s, 1H).

IR ($CHCl_3$): 2922, 2852, 1676, 1600, 1561, 1466 $cm^{-1}$.

EXAMPLE 5 (ILLUSTRATIVE EXAMPLE)

2-Bromo-N-(2,4,6-trimethoxyphenyl)decanamide

2-Bromodecanoic acid (1 g, 3.8 mmol) was heated under reflux in thionyl chloride (10 ml) for 1 hour. The thionyl chloride was evaporated and the residue was dissolved in dry ether (10 ml) and added dropwise to a solution of 2,4,6-trimethoxyaniline (0.7 g, 3.8 mmol) in pyridine (20 ml) at 0 °C and the mixture was stirred for 1.5 hours. The reaction mixture was poured into saturated aqueous ammonium chloride and extracted three times with ethyl acetate (60 ml). The combined organics were extracted with water and brine and dried and concentrated. Recrystallization from isopropyl ether afforded 1.1 g (65%) of the title compound, m.p. 109-110 °C. This material was used directly in the next step.

EXAMPLE 6 (ILLUSTRATIVE EXAMPLE)

N-(2,4,6-Trimethoxy)phenyl-2-((2-pyridyl)thio)decanoamide

2-Thiopyridine (0.27 g, 2.4 mmol) in dimethylformamide (20 ml) was treated with sodium hydride (0.1 g, 2.4 mmol, 60% oil dispersion) and stirred for 15 minutes at 25 °C. To this cloudy solution, the title compound of Example 5 (1.0 g, 2.4 mmol) in dimethylformamide (10 ml) was added and the mixture and

was stirred at 25°C for 1.5 hours. The reaction mixture was then poured into 1 N HCl (75 ml) and extracted 3 times with ethyl acetate (125 ml). The organics were dried, concentrated and chromatographed on silica gel (eluted with 1:1 ethyl acetate:hexanes). Recrystallization from isopropyl ethyl afforded 0.4 g (36%) of the title compound, m.p. 92°C.

$^1$H NMR ($CDCl_3$): δ 8.52-8.42 (m, 2H), 7.54 (t, J=4 Hz, 1H), 7.26 (m, 1H), 7.04 (t, J=4 Hz, 1H), 6.12 (s, 2H), 4.53 (t, J=3 Hz, 1H), 3.80 (s, 3H), 3.66 (s, 6H),
Anal. Calculated for $C_{24}H_{34}O_4N_2S$: C, 64.55; H, 7.67; N, 6.27. Found: C, 64.34; H, 7.54; N, 6.20.

EXAMPLE 7 (ILLUSTRATIVE EXAMPLE)

N-(Isoquinolin-5-yl)-2-((2-pyridyl)thio) decanamide

This compound was prepared by a procedure similar to that described in Example 4.

M.p. 81-83°C. $^1$H NMR: δ 9.24 (bs, 1); 8.60 - 8.36 (m, 3); 7.78 - 7.11 (m, 7); 4.53 (t, 3 Hz, 1); ($CHCl_3$): 2940, 2860, 1700, 1590 cm$^{-1}$.

EXAMPLE 8 (ILLUSTRATIVE EXAMPLE)

N-(2,4,6-Trimethoxyphenyl)-2-methyl-2-(4-(1-methylpropyl)phenoxy)nonanoic amide

By use of the procedures described in Examples 1 and 3, ethyl 2-bromononanoate and 4-(1-methylpropyl)phenol were coupled and the product saponified to give 2-(4-(1-methylpropyl)phenoxy)-nonanoic acid. This material (1.0 g) was then methylated at the 2-position according to the procedure of Pfeffer, et. al. (J. Org. Chem., 1972, 37, 451) to give 2-methyl-2-hexanethiodecanoic acid (0.928 g). This material (0.86 g) was converted to the corresponding acid chloride with oxalyl chloride and coupled with 2,4,6-trimethoxyaniline (0.49 g) according to the procedure of Adams and Ulrich (J. Am. Chem. Soc., 1920, 42, 599) to give the title compound (1.12 g).

Oil. $^1$H NMR: δ 7.82 (s, 1H); 7.12 (d, 6 Hz, 2 H); 7.07 (d, 6 Hz, 2 H); 6.19 (s, 2 H); 3.85 (s, 3 H); 3.83 (s, 6 H); 2.61 (dt, 8 Hz, 1 H); 1.98 (m, 2 H); 1.68 - 1.20 (m, 12 H); 1.52 (s, 3 H); 1.26 (d, 8 Hz, 3 H); 0.93 (m, 3 H); 0.85 (t, 8 Hz, 3 H). $^{13}$C NMR: δ 173.38, 159.90, 156.48, 152.69, 142.12, 127.39, 121.24, 107.15, 91.02, 84.41, 55.83, 55.47, 40.94, 40.06, 31.86, 31.32, 29.85, 29.33, 23.46, 22.68, 21.90, 21.67, 14.11, 12.21.
IR ($CHCl_3$) cm$^{-1}$: 3410, 2940, 2850, 1680, 1608. Mass spectrum m/e (relative intensity): M+ 485.42 (16), 336.28 (33), 308.28 (24), 275.30 (30), 209.04 (40), 183.14 (100). High resolution mass spectra: m.e 485.3134, calcd for $C_{29}H_{43}NO_5$: 485.3141. Anal.: Calc'd for $C_{29}H_{43}NO_5$: C, 71.72; H, 8.93; N, 2.88. Found: C, 71.28; H, 8.87; N, 2.74.

EXAMPLE 9

N-(6-methylthioquinolin-5-yl)-2-(hexylthio)decanoic amide

Commercially available 6-chloroquinoline (33.3 g) was nitrated according to the procedure of Campbell et al, (J. Am. Chem. Soc., 1946, 68, 1559) to give 5-nitro-6-chloroquinoline (20.36 g). This material (15 g) was allowed to react with sodium methylthiolate according to the procedure of Massie (Iowa State Coll. J. Sci. 1946, 21, 41; CA 41:3044 g) to give 5-nitro-6-methylthioquinoline (13.61 g). This material (3.70 g) was reduced using iron (5.62 g) and hydrochloric acid (1.5 ml) in 50% aqueous ethanol (50 ml) to give 5-amino-6-methylthioquinoline (3.0 g). This material (3.0 g) was coupled with 2-hexanethiodecanoic acid (5.83 g, prepared according to the procedures described in Examples 1 and 3) using the procedure described in Example 8, to give the title compound (3.8 g).

M.p. 91-92°C. $^1$H NMR: δ 8.85 (d, 3 Hz, 1 H); 8.62 (s, 1 H); 8.05 (d, 9 Hz, 1 H); 8.00 (d, 9 Hz, 1 H); 7.65 (d, 9 Hz, 1 H); 7.40 (dd, 3 & 9 Hz, 1 H); 3.55 (t, 8 Hz, 1 H); 2.80 (t, 8 Hz, 2 H); 2.50 (s, 3 H); 2.10-1.35 (m, 17 H); 0.91 (t, 9 Hz, 6 H). $^{13}$C NMR: δ 172.00, 149.84, 131.37, 129.61, 126.91, 121.76, 51.22, 33.16, 32.36, 31.91, 31.47, 29.47, 29.34, 29.30, 28.69, 27.82, 22.73, 22.59, 15.77, 14.17, 14.08. IR ($CHCl_3$) cm$^{-1}$: 3318, 2923, 2852, 1677, 1586, 1567. Mass spectrum m/e (relative intensity): M+ 460.2 (2), 413.2 (6), 344.2 (23), 295.2 (13), 243.2 (16), 217.0 (70), 190.1 (100). Anal.: Calc'd for $C_{26}H_{40}N_2OS_2$: C, 67.78; H, 8.75; N, 6.08. Found: C, 68.27; H, 8.46; N, 5.85.

EXAMPLE 10

N-(6-Methylthioquinolin-5-yl)-2-(4-sec-butylphenoxy)nonanoic amide

5-Amino-6-methylthioquinoline, prepare as described in Example 9 , was coupled with 2-(4-sec-butylphenoxy)nonanoic acid according to the procedure described in Example 8 to give the title compound.

Oil. Anal.: Found: C, 71.35; H, 7.98; N, 5.54.
Calc'd. for $C_{29}H_{38}N_2O_2S$: C, 72.76; H, 8.00; N, 5.85.

EXAMPLE 11

N-(6-Methylthioquinolin-5-yl)-2-octanyl-1,3-dithiane-2-carboxamide

5-Amino-6-methylthioquinoline, prepared as described in Example 9 , was coupled with 2-octanyl-1,3-dithiane-2-carboxylic acid, prepared by treatment of 1,3-dithiane-2-carboxylic acid with sodium hexamethyldisilazide and octanyl bromide, according to Example 8 to give the title compound.

Oil. Anal.: Found: C, 59.11; H, 5.81; N, 6.07.
Calc'd. for $C_{23}H_{32}N_2OS_3$: C, 61.57; H, 7.19; N, 6.24.

EXAMPLE 12

N-(6-(hexylthio)quinolin-5-yl)-2-hexylthiodecanoic amide

Anal: calc'd for $C_{31}H_{50}N_2OS_2$: C, 70.13; H, 9.49; N, 5.28. Found: C, 69.99; H, 9.37; N, 5.42.

EXAMPLE 13

N-(6-methylthioquinolin-5-yl)oleamide

5-Amino-6-methylthioquinoline, prepared as described in Example 9 , and commercially available oleoyl chloride were coupled to give the title compound according to the procedure described in Example 8. Mass spectrum m/e: 454.3 (M+).

EXAMPLE 14

N-(6-methylthioquinolin-5-yl)-2,2-di(hexylthio)acetamide

2,2-Di(hexylthio)acetic acid was synthesized from dichloroacetic acid and hexanethiol using a procedure similar to that described in Example 1. 2,2-Di(hexylthio)acetic acid and 5-amino-6-methylthioquinoline were coupled to give the title compound using the procedure described in Example 8.

Mass spectrum m/e: 464.2 (M+).

$^1$H NMR δ ($CDCl_3$): 8.83 (dd, J=3 & 4 Hz, 1H); 8.45 (s, 1H), 8.12 (d, J=8 Hz, 1H); 8.09 (d, J=8 Hz, 1H); 7.64, d, J=8Hz, 1 H); 7.42 (dd, J=4 & 8, 1H); 4.51 (s, 1H); 2.89 (t, J=8 Hz, 4H); 2.57 (s, 3H), 1.68 (m, 6H), 1.44 (m, 6H); 1.32 (m, 8H); 0.81 (t, J=7 Hz, 6H).

EXAMPLE 15

N-(6-methylthioquinolin-5-yl)-2-heptylnonanoic amide

By use of the procedures described in Example 8, nonanoic acid was alkylated with heptyl bromide and the resulting product was coupled with 5-amino-6-methylthioquinoline (Example 9) to give the title compound.

Mass spectrum m/e: 428.3 (M+ - $SCH_3$).

$^1$H NMR ($CDCl_3$): δ 8.77 (d, J=4 Hz, 1H); 8.00 (d, J=8 Hz, 1H); 7.95 (d, J=8 Hz, 1H); 7.55 (d, J=8 Hz, 1H); 7.49 (s, 1H); 7.31 (dd, J=4 & 8 Hz, 1H); 2.49 (s, 3H); 2.43 (m, 1H); 1.40 (br, 24H); 0.85 (t, J=6 Hz, 6H).

EXAMPLE 16

N-(6-methylthioquinolin-5-yl)-2-[2-(6-ethoxybenzthiazolyl)-thio]decanoic amide.

The title compound was prepared by procedures analogous to those as described in Examples 5 and 6.

MP: 139-141 °C

$^1$H NMR ($CDCl_3$): δ 9.42 (s, 1H); 8.76 (d, 3Hz, 1H); 8.00 (d, 9Hz, 2H); 7.74 (d, 9Hz, 1H); 7.60 (d, 9Hz, 1H); 7.21 (m, 2H); 6.98 (dd, 3 & 9 Hz, 1H); 4.71 (t, 7Hz, 1H); 4.05 (q, 7 Hz, 2H); 2.53 (s, 3H); 2.30 (m, 1H); 1.97 (m, 1H); 1.59 (m, 2H); 1.45-1.25 (m, 10H); 1.00-0.81 (m, 6H).

FABMS m/e: 554 ($M^+ + 1$)

Anal.: Calc'd for $C_{29}H_{35}N_3O_2S_3 \cdot 1/2\ H_2O$: C, 61.89; H, 6.45; N, 7.46.

Found: C, 62.14; H, 6.33; N, 7.43.

EXAMPLE 17

N-(6-methylthiophthalazin-5-yl) oleic amide

5-Nitro-6-methylthiophthalazine, prepared as described by Sturrock et al., Can. J. Chem., 49, 3047 (1971) and Hirsch & Orphanos, J. Heterocyclic Chem., 2, 206 (1965), was reduced with tin(II) chloride and HCl to yield 5-amino-6-methylthiophthalazine. This material was coupled with commercially available oleoyl chloride to yield the title compound.

M.p.: oil

$^1$H NMR ($CDCl_3$): δ 9.34 (s,2H); 7.74 (d, 8Hz, 1H); 7.73 (s, 1H); 7.66 (d, 8Hz, 1H); 5.34 (t, 5Hz, 2H); 2.57 (t, 8 Hz, 2H); 2.54 (s, 3H); 2.01 (m, 4H); 1.81 (m, 2H); 1.50-1.10 (m, 20H); 0.86 (t, 6Hz, 3H).

FABMS m/e: 456 ($M^+ + 1$)

EXAMPLE 18

N-(6-methylthioquinolin-5-yl)-2,2-dimethyldodecanoic amide

The title compound was prepared in 4% yield according to the procedure of Example 4B.

$^1$H NMR ($CDCl_3$): δ 0.86 (t, 3H); 1.2-1.78 (c) and 1.42 (s)(total 24H); 2.55 (s, 3H); 7.44 (m, 2H); 7.66 (d, 1H); 8.07 (d, 1H); 8.13 (d, 1H); 8.83 (m, 1H).

IR ($CHCl_3$): 2921, 2851, 1677, 1565, 1463, 1375 $cm^{-1}$.

EXAMPLE 19

N-(6-methylthioquinolin-5-yl)tetradecanoic amide

The title compound was prepared on 31% yield according to the procedure of Example 4B.

$^1$H NMR ($CDCl_3$): δ 0.87 (t, 3H); 1.2-1.6 (c, 20H); 1.84 (m, 2H); 2.54 (s) and 2.55 (t)(total 5H); 7.18 (s, 1H); 7.40 (m, 1H); 7.64 (d, 1H); 8.06 (m, 2H); 8.84 (b, 1H).

IR ($CHCl_3$): 2919, 2849, 1683, 1365, 1464, 1377 $cm^{-1}$.

EXAMPLE 20

N-(6-methylthioquinolin-5-yl)-2-nonyl-1,2,3,4-tetrahydro-2-naphthoic amide

The title compound was prepared in 3% yield according to the procedure of Example 4.

$^1$H NMR ($CDCl_3$): δ 0.87 (t, 3H); 1.16-2.06 (c, 17H); 2.38 (m, 1H); 2.47 (s, 3H); 2.85-3.15 (c, 3H); 3.35 (d, 1H); 7.18 (m, 5H); 7.37 (s, 1H); 7.47 (d, 1H); 7.59 (d, 1H), 7.99 (d, 1H); 8.77 (s, 1H).

EXAMPLE 21

(2S)-N-[6-(methylthio)quinolin-5-yl]-2-hexylthiodecanoic amide

The title compound was prepared by the recoupling of S-(-)-2-hexylthio-decanoic acid with 5-amino-6-methylthioquinoline (Example 9) according to the procedure of Example 8. M.p.: 114-115 °C.

Optical rotation: $[\alpha]_D^{23}$ = -52° (in $CDCl_3$). Analytical analysis of enantiomeric purity was accomplished using a Chiracel OD HPLC column.

EXAMPLE 22

N-(6-methylthioquinolin-5-yl)-2-bromodecanamide

To a stirred solution of 2-bromodecanoic acid (184 mg, 0.73 mmol) in $CH_2Cl_2$ (3.0 ml) was injected by syringe oxalyl chloride (0.06 ml, 105 mol %) and then DMF (1 drop). After stirring at room temperature for 1 hour, N-methyl morpoline (0.24 ml, 300 mol %) was added. To this mixture was injected a solution of 5-amino-6-methylthioquinoline (139 mg, 100 mol%) in $CH_2Cl_2$ (2.0 ml). After stirring at room temperature for an additional 30 min. the reaction mixture was diluted with $CH_2Cl_2$ (30 ml), poured over 1.0 M $H_3PO_4$ (100 ml), and extracted with $CH_2Cl_2$ (3 x 30 ml). The combined extracts were dried ($Na_2SO_4$), evaporated, and chromatographed using 1% triethylamine:ethyl acetate as eluants to give the title compound (192 mg, 62% yield).

IR ($CDCl_3$) 3350, 2930-2820, 1710 $cm^{-1}$.

$^1$H NMR ($CDCl_3$) $\delta$ 8.84 (m, 1H); 8.05 (m, 3H); 7.63 (d, 1H, J = 9.4 Hz); 7.41 (dd, 1H, J = 3.7, 8.5 Hz); 4.57 (dd, 1H, J = 5.5, 8.3 Hz); 2.54 (s, 3H); 2.25 (m, 1H); 2.15 (m, 1H); 1.58 (m, 2h); 1.26 (m, 10H); 0.85 (m, 3H).

EXAMPLE 23

N-(6-methylthioquinolin-5-yl)-2-hexylaminodecanamide

A mixture of N-(6-methylthioquinolin-5-yl)-2-bromodecanamide (200 mg, 0.47 mmol) and n-hexylamine (10 ml) was heated at 120°C for 1 hour, cooled to room temperature, and chromatographed using 1:24:25/triethylamine:ethyl acetate: hexane as eluants to give the title compound as a pink oil (184 mg, 88% yield).

IR ($CHCl_3$) 3280, 2940-2860, 1680 cm.

$^1$H NMR ($CDCl_3$) $\delta$ 9.38 (s, 1H); 8.80 (m, 1H); 7.97 (m, 2H); 7.62 (d, 1H, J = 8.8 Hz); 7.36 (dd, 1H, J = 4.3, 8.6Hz); 3.28 (dd, 1H, J = 4.7, 8.6 Hz); 2.86 (m, 1H), 2.78 (m, 1H); 2.51 (s, 3H); 1.98 (m, 1H); 1.74 (m, 1H); 1.56 (m, 5H); 1.30 (m, 16H); 0.86 (m, 6H). Mass spectum m/e (relative intensity): $M^+$ 444.30 (82), 226.40 (100).

Anal. Calc'd for $C_{26}H_{41}N_3OS$: C, 70.4; H, 9.3; N, 9.5;

Found: C, 70.9; H, 9.4; N, 9.5.

EXAMPLE 24

N-(6-methylthioquinolin-5-yl)-2-N,N-[(acyl)(hexyl)]aminodecanamide

To a stirred solution of N-(6-methylthioquinolin-5-yl)-2-hexylaminodecanamide (200 mg, 0.45 mmol) pyridine (10 ml) was added in one portion acetic anhydride (2.0 ml). After stirring at room temperature for 1 hour, the mixture was poured over 1.0 M $H_3PO_4$ (200 ml) and extracted with $CH_2Cl_2$ (3 x 100 ml). The combined extracts were dried ($Na_2SO_4$), evaporated, and chromatographed using ethyl acetate as eluant to give the title compound (200 mg, 91% yield).

$^1$H NMR ($CDCl_3$) $\delta$ 8.81 (d, 1H, J = 2.9 Hz); 8.66 (s, 1H); 8.01 (d, 1H, J = 6.7 Hz); 8.00 (d, 1H, J = 2.0 Hz); 7.60 (d, 1H, J = 9.1 Hz); 7.36 (dd, 1H, J = 4.2, 8.6 Hz); 4.95 (m, 1H), 3.33 (t, 2H, J = 8.3 Hz); 2.50(s, 3H); 2.24 (s, 3H); 2.15 (m, 1H); 1.88 (m, 1H); 1.66 (m, 2H); 1.25 (m, 18H); 0.86 (m, 6H).

Mass spectrum m/e (relative intensity): $M^+$ 486.3(87), 296.3 (62), 268.3(32), 226.3 (100).

EXAMPLE 25

N-(6-methylthioquinolin-5-yl)-2-N,N-[(hexyl)(methylsulfonyl)]aminodecanamide

To a stirred solution of N-(6-methylthioquinolin-5-yl)-2-hexylaminodecanamide (200 mg, 0.45 mmol) and triethylamine (0.19 ml, 300 mol%) in $CH_2Cl_2$ (5 ml) was added dropwise a mixture of methanesulfonyl chloride (0.038 ml, 110 mol%) in $CH_2Cl_2$ (5 ml) at 0°C. After stirring at 0°C for 30 min, the reaction mixture was allowed to warm to room temperature. After stirring at room temperature for 3 hours, the

mixture was chromatographed using 1% triethylamine:ethyl acetate as eluants to give the title compound (120 mg, 51% yield).

$^1$H NMR ($CDCl_3$) δ 8.84 (s, 1H); 8.31 (s, 1H); 8.13 (d, 1H, J = 8.4 Hz); 8.05 (d, 1H, J = 9.0 Hz); 7.64 (d, 1H, J = 9.0 Hz); 7.42 (dd, 1H, J = 4.2, 8.5 Hz); 4.51 (t, 1H, J = 7.4 Hz); 3.40 (m, 3H); 3.01 (s, 3H); 2.54 (s, 3H); 2.19 (m, 1H); 1.77 (m, 2H); 1.28 (m, 18H); 0.87 (m, 6H).

Mass spectrum m/e (relative intensity): $M^+$ 522.3 (100).

EXAMPLE 26

N-(6-methylthioquinolin-5-yl)-2-N,N-[(benzenesulfonyl)(hexyl)]aminodecanamide

The title compound was prepared according to the procedure of Example 25.

$^1$H NMR ($CDCl_3$) δ 8.85 (s, 1H); 8.50 (s, 1H); 8.22 (d, 1H, J = 8.4 Hz); 8.11 (d, 1H, J = 9.0 Hz); 7.91 (m, 2H); 7.68 (d, 1H, J = 9.0 Hz); 7.59 (m, 3H); 7.45 (dd, 1H, J = 4.1, 8.5 Hz); 4.45 (t, 1H, J = 7.3 Hz); 3.49 (m, 2H); 3.28 (m, 1H); 2.56 (s, 3H); 2.06 (m, 1H); 1.76 (m, 2H); 1.20 (m, 18H); 0.86 (m, 6H).

Mass spectrum m/e (relative intensity): $M^+$ 584.4 (100).

EXAMPLE 27

N-(6-methylthioisoquinolin-5-yl)-2-(hexylthio)decanamide

Commercially available 4-chlorobenzaldehyde (10 g) was cyclized with aminoacetaldehyde diethyl acetal according to the procedure of Hendrickson, et. al., J. Org. Chem., 48, 3344 (1983), to give 6-chloroisoquinoline (600 mg). Nitration of the obtained product using the procedure of Campbell et. al., J. Am. Chem. Soc., 68, 1559 (1946) gave 6-chloro-5-nitroisoquinoline (700 mg). Substitution of 6-chloro for thiomethoxide according to the procedure of Massie, Iowa State Coll. J. Sci., 21, 41 (1946) (Ca 41 : 3033 g) gave 6-methylthio-5-nitroisoquinoline (632 mg). This material (200 mg) was reduced using stannous chloride (2.0 g) and concentrated hydrochloric acid (30 ml) to give 5-amino-6-(methylthio)isoquinoline (143 mg). This material (140 mg) was coupled with 2-hexylthiodecanoic acid (prepared according to the procedures described in Examples 1 and 3) using the procedure described in Example 8 to give the title compound (80 mg).

$^1$H NMR ($CDCl_3$) δ 9.16 (s, 1H); 8.59 (s, 1H); 8.48 (d, (1H, J = 5.9 Hz); 7.88 (d, 1H, J = 8.7 Hz); 7.48 (m, 2H); 3.54 (dd, 1H, J = 6.3, 8.1 Hz); 2.79 (t, 2H, J = 7.4Hz); 2.55 (s, 3H); 2.12 (m, 1H); 1.88 (m, 1H); 1.65 (m, 4H); 1.30 (m, 16 H); 0.88 (m, 6H).

Mass spectrum m/e (relative intensity): $M^+$ 461.3;

Anal. calc'd for $C_{26}H_{40}N_2OS_2$: C, 67.8; H, 8.8; N, 6.1;

Found: C, 67.9; H, 8.9; N, 6.1.

EXAMPLE 28

N-(4-methylthioisoquinolin-5-yl)-2-hexylthiodecanamide

5-Amino-4-bromoisoquinoline (prepared according to the process described by Gordon et al., J. Het. Chem., 4, 410 (1967)) was allowed to react with sodium thiomethoxide according to the procedure of Massie, Iowa State Coll. J. Sci., 21, 41 (1946) (Ca. 41: 3044 g) to give 5-amino-4-methylthioisoquinoline. This material was coupled with 2-hexylthiodecanoic acid (prepared by the procedures described in Examples 1 and 3) using the procedure described in Example 8 to give the title compound.

IR (KBr) 3230, 3000-2800, 1660 $cm^{-1}$.

$^1$H NMR ($CDCl_3$) δ 11.65 (s, 1H); 9.12 (s, 1H); 8.84 (d, 1H, J = 7.8 Hz); 8.63 (s, 1H); 7.76 (dd, 1H, J = 1.2, 8.1 Hz); 7.64 (t, 1H, J = 8.0 Hz); 3.44 (t, 1H, J = 7.5 Hz); 2.65 (m, 2H); 2.53 (s, 3H); 2.03 (m, 1H); 1.83 (m, 1H); 1.59 (m, 4H); 1.24 (m, 16H); 0.84 (m, 6H).

Mass spectrum m/e (relative intensity): $M^+$ 461.3 (100). Anal. calc'd for $C_{26}H_{40}N_2OS_2$: C, 67.8; H, 8.8; N, 6.1. Found: C, 68.0; H, 8.9; N, 6.0.

## Claims

**Claims for the following Contracting States : AT, BE, CH, DE, DK, FR, GB, IT, LI, LU, NL, SE**

**1.** A compound of the formula

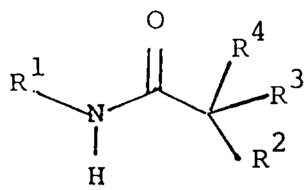

I

wherein $R^1$ is:

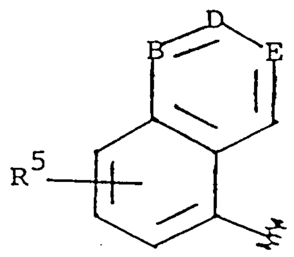

XXIV

$R^2$, $R^3$ and $R^4$ may be the same or different, and

(a) are selected from the group consisting of hydrogen, $(C_1\text{-}C_4)$ alkyl, A, $XR^{10}$, phenyl-$(C_1\text{-}C_7)$ alkyl, and $(C_5\text{-}C_6)$ cycloalkyl-$(C_1\text{-}C_6)$ alkyl, with the proviso that at least one of $R^2$, $R^3$ and $R^4$ must be A; or

(b) $R^2$ and $R^3$ together with the carbon to which they are attached form a cyclic or bicyclic system selected from the group consisting of $(C_3\text{-}C_7)$ cycloalkyl, $(C_3\text{-}C_7)$ cycloalkenyl, $(C_6\text{-}C_{14})$ bicycloalkyl, $(C_6\text{-}C_{14})$ bicycloalkenyl, and aryl-fused and heteroaryl-fused systems containing 8 to 15 carbon atoms, one ring of any of said aryl-fused and heteroaryl-fused systems being aromatic and the ring containing the carbon to which $R^2$ and $R^3$ are attached being non-aromatic, one of the carbons of said aromatic ring being optionally replaced by sulfur or oxygen, one or more carbons of said non-aromatic ring being optionally replaced by sulfur or oxygen, and one or more carbons of said aromatic ring being optionally replaced by nitrogen; one or two carbons of said cycloalkyl or bicycloalkyl groups being optionally replaced by sulfur or oxygen, and said cyclic or bicyclic system being optionally substituted with one to five substituents independently selected from the group consisting of phenyl, substituted phenyl, $(C_1\text{-}C_6)$ alkyl and A, with the proviso that one and only one of said substituents is A, and one and only one of said substituents is phenyl or substituted phenyl, said substituted phenyl being substituted with one or more substituents independently selected from the group consisting of $(C_1\text{-}C_6)$ alkyl, $(C_1\text{-}C_6)$ alkylthio, halogen and trifluoromethyl; and $R^4$ is hydrogen; $XR^{10}$ or A;

A is a hydrocarbon containing 4 to 16 carbons and 0, 1 or 2 double bonds:

X is O, S, SO, $SO_2$, NH, $NR^{23}CO$ or $NSO_2R^{24}$, wherein $R^{23}$ is hydrogen or $(C_1\text{-}C_6)$ alkyl and $R^{24}$ is $(C_1\text{-}C_6)$ alkyl, phenyl or $(C_1\text{-}C_3)$ alkyl-phenyl:

$R^5$ is $(C_1\text{-}C_6)$ alkylthio, which may be attached to either ring of the bicyclic ring system.

$R^{10}$ is selected from the group consisting of $(C_4\text{-}C_{12})$ cycloalkyl, $(C_4\text{-}C_{12})$ straight or branched alkyl, $(C_4\text{-}C_{12})$ cycloalkyl-$(C_1\text{-}C_6)$ alkyl, phenyl-$(C_1\text{-}C_6)$ alkyl, (substituted phenyl)-$(C_1\text{-}C_6)$ alkyl, $(C_1\text{-}C_6)$ alkyl-phenyl, $(C_1\text{-}C_6)$ alkyl-(substituted phenyl), substituted thiazoles substituted benzothiazoles, and substituted pyridines; wherein the substituents on the substituted phenyl, substituted thiazoles, substituted benzothiazoles and substituted pyridines are selected from the group consisting of $(C_1\text{-}C_4)$ alkoxy, $(C_1\text{-}C_4)$ alkylthio, $(C_1\text{-}C_6)$ alkyl, halo and trifluoromethyl;

B, D and E are selected from the group consisting of nitrogen and carbon, with the proviso that one or

two of B, D and E is nitrogen.

**2.** A compound according to claim 1 wherein $R^1$ is 6-methylthioquinolin-5-yl or 6-methylthioisoquinolin-5-yl.

**3.** A compound according to claim 1, said compound being selected from the group consisting of:
N-(6-methylthioquinolin-5-yl)-2-(hexylthio)decanoic amide;
N-(6-methylthioisoquinolin-5-yl)-2-(hexylthio)decanoic amide;
N-(6-methylthioquinolin-5-yl)-2-(4-(3-methylpropyl)phenoxy)nonanoic amide;
(2S)-N-[6-(methylthio)quinolin-5-yl]-2-hexylthiodecanoic amide; and
N-(6-methylthioquinolin-5-yl)-2-octyl-1,3-dithian-2-yl carboxamide.

**4.** A pharmaceutical composition for inhibiting acyl coenzyme A: cholesterol acyltransferase, inhibiting intestinal absorption of cholesterol, reversing or slowing the development of atherosclerosis, or lowering the concentration of serum cholesterol in a mammal, comprising an amount of a compound according to claim 1 that is effective in inhibiting acyl coenzyme A: cholesterol acyl-transferase or intestinal absorption of cholesterol, or is effective in reversing or slowing the development of atherosclerosis or lowering the concentration of serum cholesterol, and a pharmaceutically acceptable carrier.

**5.** A compound according to claim 1, wherein:
(a) $R^4$ is A, and $R^2$ and $R^3$ together with the carbon to which they are attached form a cyclic or bicyclic ring system selected from the group consisting of

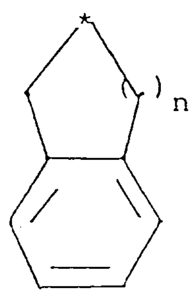

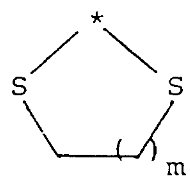

wherein n and m are each independently 1 or 2, and the asterisk designates the carbon to which $R^2$ and $R^3$ are attached; or
(b) $R^4$ is A, and $R^2$ and $R^3$ together with the carbon to which they are attached form the bicyclic ring system

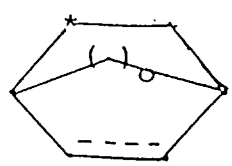

wherein o is 0, 1 or 2, and the asterisk designates the carbon to which $R^2$ and $R^3$ are attached; or
(c) $R^4$ is hydrogen, and $R^2$ and $R^3$ together with the carbon to which they are attached form a cyclic or bicyclic ring system selected from the group consisting

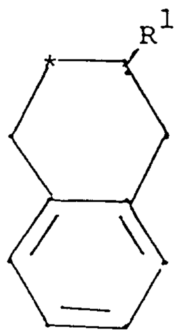

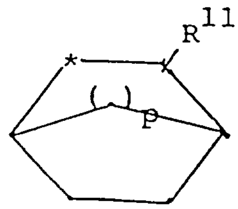

and

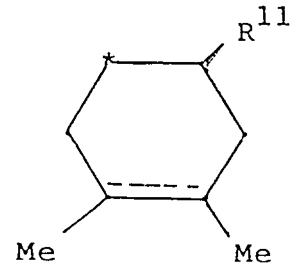

,

of wherein p is 0, 1 or 2, $R^{11}$ is A, the dotted line represents an optional double bond, and the asterisk designates the carbon to which $R^2$ and $R^3$ are attached.

**Claims for the following Contracting States : ES, GR**

**1.** A process for making compound of the formula

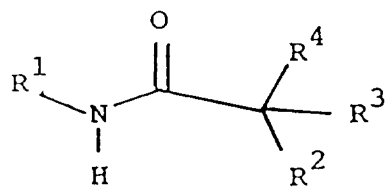

I

wherein $R^1$ is

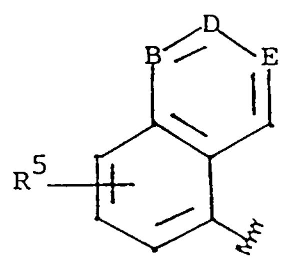

XXIV

$R^2$ $R^3$ and $R^4$ may be the same or different, and

(a) are selected from the group consisting of hydrogen, $(C_1\text{-}C_4)$ alkyl, A, $XR^{10}$, phenyl-$(C_1\text{-}C_7)$ alkyl, and $(C_5\text{-}C_6)$ cycloalkyl-$(C_1\text{-}C_6)$ alkyl, with the proviso that at least one of $R^2$, $R^3$ and $R^4$ must be A; or

(b) $R^2$ and $R^3$ together with the carbon to which they are attached form a cyclic or bicyclic system selected from the group consisting of $(C_3\text{-}C_7)$ cycloalkyl, $(C_3\text{-}C_7)$ cycloalkenyl, $(C_6\text{-}C_{14})$ bicycloalkyl, $(C_6\text{-}C_{14})$ bicycloalkenyl, and aryl-fused and heteroaryl-fused systems containing 8 to 15 carbon atoms, one ring of said aryl-fused and heteroaryl-fused systems being aromatic and the ring containing the carbon to which $R^2$ and $R^3$ are attached being non-aromatic, one of the carbons of said aromatic ring being optionally replaced by sulfur or oxygen, one or more carbons of said non-aromatic ring being optionally replaced by sulfur or oxygen, and one or more carbons of said aromatic ring being optionally replaced by nitrogen; one or two carbons of said cycloalkyl or bicycloalkyl groups being optionally replaced by sulfur or oxygen, and said cyclic or bicyclic system being optionally substituted with one to five substituents independently selected from the group consisting of phenyl, substituted phenyl, $(C_1\text{-}C_6)$ alkyl and A, with the proviso that one and only one

of said substituents is A, and one and only one of said substituents is phenyl or substituted phenyl, said substituted phenyl being substituted with one or more substituents independently selected from the group consisting of ($C_1$-$C_6$) alkyl, ($C_1$-$C_6$) alkylthio, halogen and trifluoromethyl; and $R^4$ is hydrogen, $XR^{10}$ or A;

A is a hydrocarbon containing 4 to 16 carbons and 0, 1 or 2 double bonds:

X is O, S, SO, $SO_2$, NH, $NR^{23}CO$ or $NSO_2R^{24}$, wherein $R^{23}$ is hydrogen or ($C_1$-$C_6$) alkyl and $R^{24}$ is ($C_1$-$C_6$) alkyl, phenyl or ($C_1$-$C_3$) alkyl-phenyl:

$R^5$ is ($C_1$-$C_6$) alkylthio, which may be attached to either ring of the bicyclic ring system.

$R^{10}$ is selected from the group consisting of ($C_4$-$C_{12}$) cycloalkyl, ($C_4$-$C_{12}$) straight or branched alkyl, ($C_4$-$C_{12}$) cycloalkyl-($C_1$-$C_6$) alkyl, phenyl-($C_1$-$C_6$) alkyl, (substituted phenyl)-($C_1$-$C_6$) alkyl, ($C_1$-$C_6$) alkyl-phenyl, ($C_1$-$c_6$) alkyl-(substituted phenyl), substituted thiazoles, substituted benzothiazoles, and substituted pyridines; wherein the substituents on the substituted phenyl, substituted thiazoles, substituted benzothiazoles and substituted pyridines are selected from the group consisting of ($C_1$-$C_4$) alkoxy, ($C_1$-$C_4$) alkylthio, ($C_1$-$C_6$) alkyl, halo and trifluoromethyl:

B, D and E are selected from the group consisting of nitrogen and carbon, with the proviso that one or two of B, D and E is nitrogen,

or a pharmaceutically acceptable salt thereof, comprising:

reacting a compound of the formula

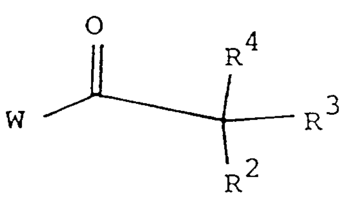

IV

wherein W is chloro or bromo and $R^2$, $R^3$ and $R^4$ are defined as above, with an amine of the formula $R^1NH_2$, wherein $R^1$ is defined as above, and an acid scavenger.

**2.** A process according to claim 1, wherein said compound of the formula IV is obtained by reacting a compound of the formula

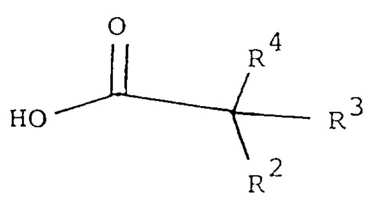

III

wherein $R^2$, $R^3$ and $R^4$ are defined as in claim 1, with a chlorinating or brominating agent.

**3.** A process according to claim 1 and claim 2, wherein said acid scavenger is selected from the group consisting of dimethylaminopyridine, pyridine and triethylamine, and said chlorinating or brominating agent is selected from the group consisting of oxalyl chloride, oxalyl bromide, thionyl chloride, thionyl bromide, phosphorus trichloride, phosphorous tribromide, phosphorous pentachloride, phosphorous pentabromide, phosphorous oxychloride and phosphorous oxybromide.

## Patentansprüche

## Patentansprüche für folgende Vertragsstaaten : AT, BE, CH, DE, DK, FR, GB, IT, LI, LU, NL, SE

**1.** Verbindung der Formel

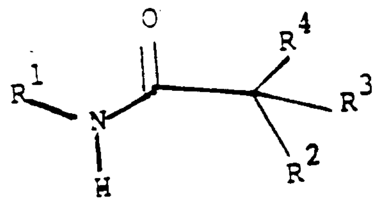

I,

worin $R^1$

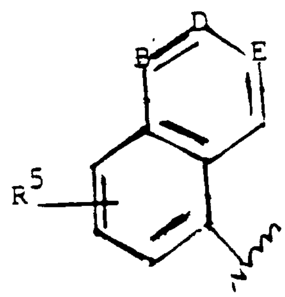

XXIV

ist, $R^2$, $R^3$ und $R^4$ gleich oder verschieden sein können und

(a) aus der aus Wasserstoff, $(C_1\text{-}C_4\text{-})$Alkyl, A, $XR^{10}$, Phenyl-$(C_1\text{-}C_7)$-alkyl und $(C_5\text{-}C_6)$-Cycloalkyl-$(C_1\text{-}C_6)$-alkyl bestehenden Gruppe ausgewählt werden, mit der Maßgabe, daß mindestens einer der Substituenten $R^2$, $R^3$ und $R^4$ A sein muß, oder

(b) $R^2$ und $R^3$ zusammen mit dem Kohlenstoffatom, an welches sie gebunden sind, ein cyclisches oder bicyclisches System, ausgewählt aus der aus $(C_3\text{-}C_7)$-Cycloalkyl, $(C_3\text{-}C_7)$-Cycloalkenyl, $(C_6\text{-}C_{14})$-Bicycloalkyl, $(C_6\text{-}C_{14})$-Bicycloalkenyl und aryl-kondensierten und heteroaryl-kondensierten Systemen mit 8 bis 15 Kohlenstoffatomen bestehenden Gruppe, bilden, wobei ein Ring eines dieser aryl-kondensierten und heteroaryl-kondensierten Systeme aromatisch ist und der Ring, der das Kohlenstoffatom, an welches $R^2$ und $R^3$ gebunden sind, trägt, nicht-aromatisch ist, wobei eines der Kohlenstoffatome dieses aromatischen Ringes wahlweise durch Schwefel oder Sauerstoff ersetzt sein kann, ein oder mehrere Kohlenstoffatome dieses nicht-aromatischen Ringes wahlweise durch Schwefel oder Sauerstoff ersetzt sein können und ein oder mehrere Kohlenstoff-atome dieses aromatischen Ringes wahlweise durch Stickstoff ersetzt sein können; ein oder zwei Kohlenstoffatome dieser Cycloalkyl- oder Bicycloalkylgruppen wahlweise durch Schwefel oder Sauerstoff ersetzt sein können und das cyclische oder bicyclische System wahlweise mit 1 bis 5 Substituenten substituiert sein kann; die unabhängig aus der aus Phenyl, substituiertem Phenyl, $(C_1\text{-}C_6)$-Alkyl und A bestehenden Gruppe ausgewählt werden, mit der Maßgabe, daß einer und nur einer dieser Substituenten A ist und einer und nur einer dieser Substituenten Phenyl oder substituiertes Phenyl ist, wobei das substituierte Phenyl mit einem oder mehreren Substituenten substituiert sein kann, die unabhängig aus der aus $(C_1\text{-}C_6)$-Alkyl, $(C_1\text{-}C_6)$-Alkylthio, Halogen und Trifluormethyl bestehenden Gruppe ausgewählt werden; und $R^4$ Wasserstoff, $XR^{10}$ oder A ist;

A einen Kohlenwasserstoff bedeutet, der 4 bis 16 Kohlenstoffatome und 0, 1 oder 2 Doppelbindungen enthält;

X O, S, SO, $SO_2$, NH, $NR^{23}CO$ oder $NSO_2R^{24}$ bedeutet, worin $R^{23}$ Wasserstoff oder $(C_1\text{-}C_6)$-Alkyl bedeutet und $R^{24}$ $(C_1\text{-}C_6)$-Alkyl, Phenyl oder $(C_1\text{-}C_3)$-Alkylphenyl bedeutet;

$R^5$ $(C_1\text{-}C_6)$-Alkylthio bedeutet, das an irgendeinen Ring des bicyclischen Ringsystems gebunden sein kann;

$R^{10}$ aus der Gruppe ausgewählt wird, die aus $(C_4\text{-}C_{12})$-Cycloalkyl, geradem oder verzweigtem $(C_4\text{-}C_{12})$-Alkyl, $(C_4\text{-}C_{12})$-Cycloalkyl-$(C_1\text{-}C_6)$-alkyl, Phenyl-$(C_1\text{-}C_6)$-alkyl, (substit.-Phenyl)-$(C_1\text{-}C_6)$-alkyl,

($C_1$-$C_6$-)Alkylphenyl, ($C_1$-$C_6$)-Alkyl-(substit.-phenyl), substituierten Thiazolen, substituierten Benzothiazolen und substituierten Pyridinen besteht, worin die Substituenten an dem substituierten Phenyl, den substituierten Thiazolen, substituierten Benzothiazolen und substituierten Pyridinen aus der aus ($C_1$-$C_4$)-Alkoxy, ($C_1$-$C_4$)-Alkylthio, ($C_1$-$C_6$)-Alkyl, Halogen und Trifluormethyl bestehenden Gruppe ausgewählt werden;

B, D und E aus der aus Stickstoff und Kohlenstoff bestehenden Gruppe ausgewählt werden, mit der Maßgabe, daß eines oder zwei der Symbole B, D und E Stickstoff bedeuten.

**2.** Verbindung nach Anspruch 1, in der $R^1$ 6-Methylthiochinolin-5-yl oder 6-Methylthioisochinolin-5-yl bedeutet.

**3.** Verbindung nach Anspruch 1, die aus der aus
N-(-6-Methylthiochinolin-5-yl)-2-(hexylthio)caprinamid,
N-(6-Methylthioisochinolin-5-yl)-2-(hexylthio)caprinamid,
N-(6-Methylthiochinolin-5-yl)-2-(4-(3-methylpropyl)phenoxy)pelargonamid,
(2S)-N-[6-(Methylthio)chinolin-5-yl]-2-hexylthiocaprinamid und
N-(6-Methylthiochinolin-5-yl)-2-octyl-1,3-dithian-2-yl-carboxamid
bestehenden Gruppe ausgewählt ist.

**4.** Pharmazeutische Zusammensetzung zum Inhibieren von Acylcoenzym A: Cholesterinacyltransferase, zum Inhibieren der intestinalen Resorption von Cholesterin, zum Umkehren oder Verlangsamen der Entwicklung von Atherosklerose oder zum Senken der Konzentration von Serumcholesterin in einem Säugetier, umfassend eine Menge einer Verbindung gemäß Anspruch 1, die zum Inhibieren von Acylcoenzym A: Cholesterinacyltransferase, zum Inhibieren der intestinalen Resorption von Cholesterin, zum Umkehren oder Verlangsamen der Entwicklung von Atherosklerose oder zum Senken der Konzentration von Serumcholesterin wirksam ist, und einen pharmazeutisch annehmbaren Träger.

**5.** Verbindung nach Anspruch 1, worin

(a) $R^4$ A ist und $R^2$ und $R^3$ zusammen mit dem Kohlenstoffatom, an das sie gebunden sind, ein cyclisches oder bicyclisches Ringsystem bilden, das aus der aus

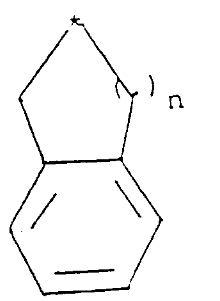

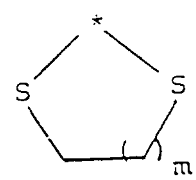

bestehenden Gruppe ausgewählt ist, worin n und m jeweils unabhängig 1 oder 2 bedeuten und das Sternchen das Kohlenstoffatom bezeichnet, an das $R^2$ und $R^3$ gebunden sind; oder

(b) $R^4$ A ist und $R^2$ und $R^3$ zusammen mit dem Kohlenstoffatom, an das sie gebunden sind, das bicyclische Ringsystem

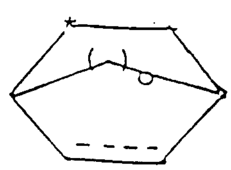

bilden, worin o 0, 1 oder 2 ist und das Sternchen das Kohlenstoffatom bezeichnet, an das $R^2$ und $R^3$ gebunden sind; oder

(c) $R^4$ Wasserstoff ist und $R^2$ und $R^3$ zusammen mit dem Kohlenstoffatom, an das sie gebunden sind, ein cyclisches oder bicyclisches Ringsystem bilden, das aus der aus

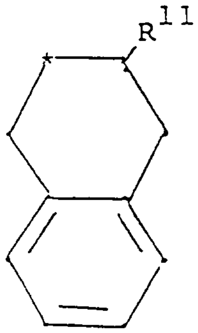 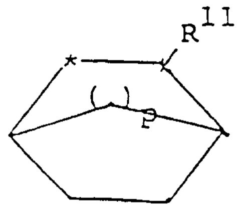 und 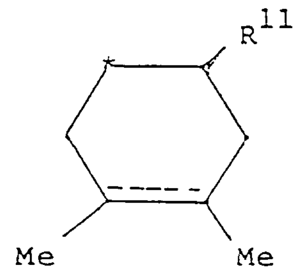

bestehenden Gruppe ausgewählt ist, worin p 0, 1 oder 2 ist, $R^{11}$ A ist, die gestrichelte Linie eine fakultative Doppelbindung bedeutet und das Sternchen das Kohlenstoffatom bezeichnet, an das $R^2$ und $R^3$ gebunden sind.

**Patentansprüche für folgende Vertragsstaaten : ES, GR**

**1.** Verfahren zur Herstellung einer Verbindung der Formel

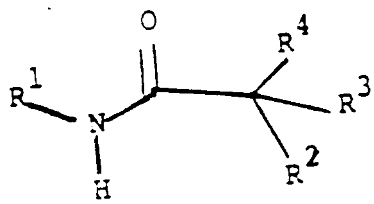 I,

worin $R^1$

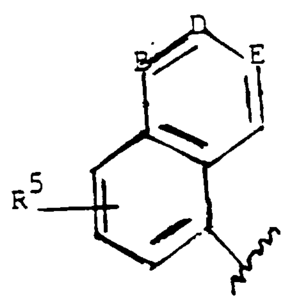

XXIV

ist, $R^2$, $R^3$ und $R^4$ gleich oder verschieden sein können und

(a) aus der aus Wasserstoff, $(C_1$-$C_4$-)Alkyl, A, $XR^{10}$, Phenyl-$(C_1$-$C_7)$-alkyl und $(C_5$-$C_6)$-Cycloalkyl-$(C_1$-$C_6)$-alkyl bestehenden Gruppe ausgewählt werden, mit der Maßgabe, daß mindestens einer der Substituenten $R^2$, $R^3$ und $R^4$ A sein muß, oder

(b) $R^2$ und $R^3$ zusammen mit dem Kohlenstoffatom, an welches sie gebunden sind, ein cyclisches oder bicyclisches System, ausgewählt aus der aus $(C_3$-$C_7)$-Cycloalkyl, $(C_3$-$C_7)$-Cycloalkenyl, $(C_6$-$C_{14})$-Bicycloalkyl, $(C_6$-$C_{14})$-Bicycloalkenyl und aryl-kondensierten und heteroaryl-kondensierten Systemen mit 8 bis 15 Kohlenstoffatomen bestehenden Gruppe, bilden, wobei ein Ring eines dieser aryl-kondensierten und heteroaryl-kondensierten Systeme aromatisch ist und der Ring, der das Kohlenstoffatom, an welches $R^2$ und $R^3$ gebunden sind, trägt, nicht-aromatisch ist, wobei eines der Kohlenstoffatome dieses aromatischen Ringes wahlweise durch Schwefel oder Sauerstoff ersetzt sein kann, ein oder mehrere Kohlenstoffatome dieses nicht-aromatischen Ringes wahlweise durch Schwefel oder Sauerstoff ersetzt sein können und ein oder mehrere Kohlenstoffatome dieses aromatischen Ringes wahlweise durch Stickstoff ersetzt sein können; ein oder zwei Kohlenstoffatome

dieser Cycloalkyl- oder Bicycloalkylgruppen wahlweise durch Schwefel oder Sauerstoff ersetzt sein können und das cyclische oder bicyclische System wahlweise mit 1 bis 5 Substituenten substituiert sein kann, die unabhängig aus der aus Phenyl, substituiertem Phenyl, $(C_1-C_6)$-Alkyl und A bestehenden Gruppe ausgewählt werden, mit der Maßgabe, daß einer und nur einer dieser Substituenten A ist und einer und nur einer dieser Substituenten Phenyl oder substituiertes Phenyl ist, wobei das substituierte Phenyl mit einem oder mehreren Substituenten substituiert sein kann, die unabhängig aus der aus $(C_1-C_6)$-Alkyl, $(C_1-C_6)$-Alkylthio, Halogen und Trifluormethyl bestehenden Gruppe ausgewählt werden; und $R^4$ Wasserstoff, $XR^{10}$ oder A ist;

A einen Kohlenwasserstoff bedeutet, der 4 bis 16 Kohlenstoffatome und 0, 1 oder 2 Doppelbindungen enthält;

X O, S, SO, $SO_2$, NH, $NR^{23}CO$ oder $NSO_2R^{24}$ bedeutet, worin $R^{23}$ Wasserstoff oder $(C_1-C_6)$-Alkyl bedeutet und $R^{24}$ $(C_1-C_6)$-Alkyl, Phenyl oder $(C_1-C_3)$-Alkylphenyl bedeutet;

$R^5$ $(C_1-C_6)$-Alkylthio bedeutet, das an irgendeinen Ring des bicyclischen Ringsystems gebunden sein kann;

$R^{10}$ aus der Gruppe ausgewählt wird, die aus $(C_4-C_{12})$-Cycloalkyl, geradem oder verzweigtem $(C_4-C_{12})$-Alkyl, $(C_4-C_{12})$-Cycloalkyl-$(C_1-C_6)$-alkyl, Phenyl-$(C_1-C_6)$-alkyl, (substit.-Phenyl)-$(C_1-C_6)$-alkyl, $(C_1-C_6$-)Alkylphenyl, $(C_1-C_6)$-Alkyl-(substit.-phenyl), substituierten Thiazolen, substituierten Benzothiazolen und substituierten Pyridinen besteht, worin die Substituenten an dem substituierten Phenyl, den substituierten Thiazolen, substituierten Benzothiazolen und substituierten Pyridinen aus der aus $(C_1-C_4)$-Alkoxy, $(C_1-C_4)$-Alkylthio, $(C_1-C_6)$-Alkyl, Halogen und Trifluormethyl bestehenden Gruppe ausgewählt werden;

B, D und E aus der aus Stickstoff und Kohlenstoff bestehenden Gruppe ausgewählt werden, mit der Maßgabe, daß eines oder zwei der Symbole B, D und E Stickstoff bedeuten

oder eines pharmazeutisch annehmbaren Salzes davon, umfassend

Umsetzen einer Verbindung der Formel

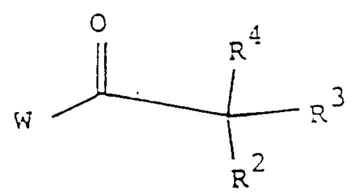

IV

worin W Chlor oder Brom ist und $R^2$, $R^3$ und $R^4$ wie oben definiert sind, mit einem Amin der Formel $R^1NH_2$, worin $R^1$ wie oben definiert ist, und einem Säurefänger.

2. Verfahren nach Anspruch 1, bei dem die Verbindung der Formel IV erhalten wird durch Umsetzen einer Verbindung der Formel

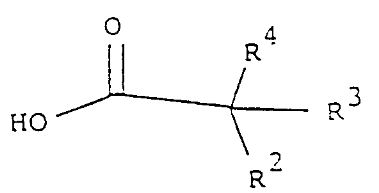

III

worin $R^2$, $R^3$ und $R^4$ wie in Anspruch 1 definiert sind, mit einem Chlorierungs- oder Bromierungsmittel.

3. Verfahren nach Anspruch 1 und Anspruch 2, bei dem der Säurefänger aus der aus Dimethylaminopyridin, Pyridin und Triethylamin bestehenden Gruppe ausgewählt wird und das Chlorierungs-oder Bromierungsmittel aus der aus Oxalylchlorid, Oxalylbromid, Thionylchlorid, Thionylbromid, Phosphortrichlorid, Phosphortribromid, Phosphorpentachlorid, Phosphorpentabromid, Phosphoroxychlorid und Phosphoroxybromid bestehenden Gruppe ausgewählt wird.

**Revendications**

**Revendications pour les Etats contractants suivants : AT, BE, CH, DE, DK, FR, GB, IT, LI, LU, NL SE**

**1.** Composé de formule

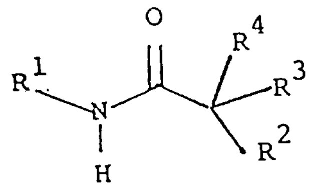

I

dans laquelle $R^1$ représente

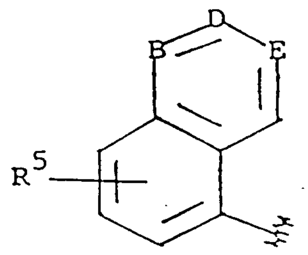

XXIV

$R^2$, $R^3$ et $R^4$ peuvent être identiques ou différents et

(a) sont choisis dans le groupe comprenant l'hydrogène, un reste alkyle en $C_1$ à $C_4$, A, $XR^{10}$, phényl(alkyle en $C_1$ à $C_7$) et (cycloalkyle en $C_5$ ou $C_6$)-(alkyle en $C_1$ à $C_6$), sous réserve que l'un au moins de $R^2$, $R^3$ et $R^4$ soit impérativement un groupe A ; ou bien

(b) $R^2$ et $R^3$ forment, conjointement avec l'atome de carbone auquel ils sont attachés, un système cyclique ou bicyclique choisi dans le groupe comprenant un reste cycloalkyle en $C_3$ à $C_7$, cycloalcényle en $C_3$ à $C_7$, bicycloalkyle en $C_6$ à $C_{14}$, bicycloalcényle en $C_6$ à $C_{14}$ et des systèmes condensés à un noyau aryle et des systèmes condensés à un noyau hétéroaryle, contenant 8 à 15 atomes de carbone, l'un des noyaux de l'un quelconque de ces systèmes condensés à un noyau aryle et condensés à un noyau hétéroaryle étant aromatique et le noyau contenant le carbone auquel $R^2$ et $R^3$ sont attachés étant non aromatique, l'un des atomes de carbone du noyau aromatique étant facultativement remplacé par du soufre ou de l'oxygène, un ou plusieurs atomes de carbone du noyau non aromatique étant facultativement remplacés par du soufre ou de l'oxygène et un ou plusieurs atomes de carbone du noyau aromatique étant facultativement remplacés par de l'azote, un ou deux atomes de carbone des groupes cycloalkyle ou bicycloalkyle étant facultativement remplacés par du soufre ou de l'oxygène, et le système cyclique ou bicyclique étant facultativement substitué avec un à cinq substituants choisis, indépendamment, dans le groupe consistant en un reste phényle, phényle substitué, alkyle en $C_1$ à $C_6$ et A, sous réserve qu'un et un seul de ces substituants soit un groupe A, et qu'un et un seul de ces substituants soit un groupe phényle ou phényle substitué, ce groupe phényle substitué portant ou plusieurs substituants choisis indépendamment dans le groupe des substituants alkyle en $C_1$ à $C_6$, alkylthio en $C_1$ à $C_6$, halogéno et trifluorométhyle ; et $R^4$ est de l'hydrogène, un groupe $XR^{10}$ ou A ;

A est un groupe hydrocarboné contenant 4 à 16 atomes de carbone et 0, 1 ou 2 doubles liaisons ;

X représente O, S, SO, $SO_2$, NH, $NR^{23}CO$ ou $NSO_2R^{24}$, où $R^{23}$ est de l'hydrogène ou un groupe alkyle en $C_1$ à $C_6$ et $R^{24}$ est un groupe alkyle en $C_1$ à $C_6$, phényle ou (alkyle en $C_1$ à $C_3$)phényle ;

$R^5$ est un groupe alkylthio en $C_1$ à $C_6$ qui peut être attaché à l'un ou l'autre des noyaux du système bicyclique.

$R^{10}$ est choisi dans le groupe comprenant un reste cycloalkyle en $C_4$ à $C_{12}$, alkyle linéaire ou ramifié en $C_4$ à $C_{12}$, (cycloalkyle en $C_4$ à $C_{12}$)-(alkyle en $C_1$ à $C_6$), phényl-(alkyle en $C_1$ à $C_6$),

(phényle substitué)-(alkyle en $C_1$ à $C_6$), (alkyle en $C_1$ à $C_6$)-phényle, (alkyle en $C_1$ à $C_6$)-(phényle substitué), des thiazoles substitués, des benzothiazoles substitués et des pyridines substituées ; les substituants du noyau phényle substitué, des thiazoles substitués, des benzothiazoles substitués et des pyridines substituées étant choisis dans le groupe des substituants alkoxy en $C_1$ à $C_4$, alkylthio en $C_1$ à $C_4$, alkyle en $C_1$ à $C_6$, halogéno et trifluorométhyle ;

B, D et E sont choisis entre de l'azote et du carbone, sous réserve qu'un ou deux de B, D et E représentent de l'azote.

2. Composé suivant la revendication 1, dans lequel $R^1$ est un reste 6-méthylthioquinoléine-5-yle ou 6-méthylthioisoquinoléine-5-yle.

3. Composé suivant la revendication 1, qui est choisi dans le groupe comprenant :
   l'amide N-(6-méthylthioquinoléine-5-yl)-2-(hexylthio)décanoïque ;
   l'amide N-(6-méthylthioisoquinoléine-5-yl)-2-hexylthio)décanoïque ;
   l'amideN-(6-méthylthioquinoléine-5-yl)-2-(4-(3-méthylpropyl)phénoxy)nonanoïque ;
   l'amide (2S)-N-[6-(méthylthio)quinoléine-5-yl]-2-hexylthiodécanoïque ; et
   le N-(6-méthylthioquinoléine-5-yl)-2-octyl-1,3-dithiane-2-yl-carboxamide.

4. Composition pharmaceutique destinée à inhiber l'acyl-coenzyme A : cholestérol-acyltransférase, à inhiber l'absorption intestinale de cholestérol, à faire rétrocéder ou à ralentir le développement de l'athérosclérose ou à abaisser la concentration du cholestérol dans le sérum chez un mammifère, comprenant une quantité d'un composé suivant la revendication 1 qui est efficace pour inhiber l'acylcoenzyme A : cholestérol-acyl-transférase ou l'absorption intestinale de cholestérol, ou qui est efficace pour faire rétrocéder ou pour ralentir le développement de l'athérosclérose ou pour abaisser la concentration du cholestérol dans le sérum, et un support acceptable du point de vue pharmaceutique.

5. Composé suivant la revendication 1, dans lequel :
   (a) $R^4$ représente A et $R^2$ et $R^3$ forment, conjointement avec l'atome de carbone auquel ils sont liés, un système cyclique ou bicyclique choisi dans le groupe constitué de :

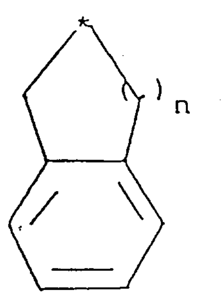

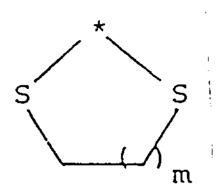

   où n et m ont chacun individuellement la valeur 1 ou 2 et l'astérisque désigne l'atome de carbone auquel $R^2$ et $R^3$ sont attachés ; ou
   (b) $R^4$ représente A et $R^2$ et $R^3$ forment conjointement avec l'atome de carbone auquel ils sont liés, le système bicyclique :

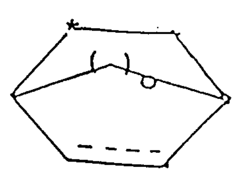

   où o a la valeur 0, 1 ou 2 et l'astérisque désigne l'atome de carbone auquel $R^2$ et $R^3$ sont liés ; ou bien

(c) $R^4$ est de l'hydrogène, et $R^2$ et $R^3$ forment, conjointement avec l'atome de carbone auquel ils sont liés, un système cyclique ou bicyclique choisi dans le groupe constitué de :

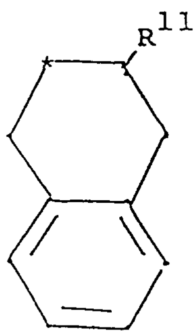 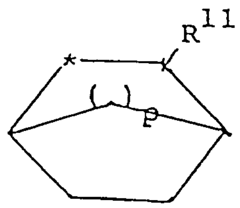 et 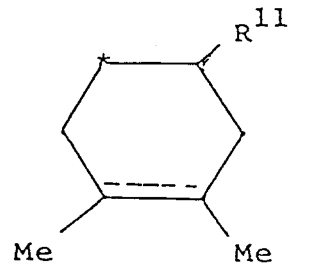 ,

où p a la valeur 0, 1 ou 2, $R^{11}$ représente A, le segment en traits interrompus représente une double liaison facultative et l'astérisque désigne l'atome de carbone auquel $R^2$ et $R^3$ sont liés.

**Revendications pour les Etats contractants suivants : ES, GR**

**1.** Procédé de production d'un composé de formule

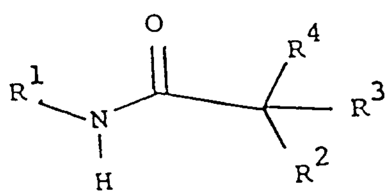

I

dans laquelle $R^1$ représente

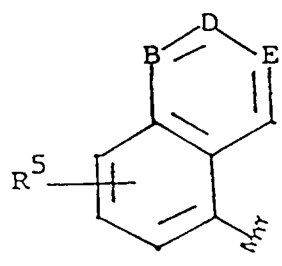

XXIV

$R^2$, $R^3$ et $R^4$ peuvent être identiques ou différents et
(a) sont choisis dans le groupe comprenant l'hydrogène, un reste alkyle en $C_1$ à $C_4$, A, $XR^{10}$, phényl-(alkyle en $C_1$ à $C_7$) et (cycloalkyle en $C_5$ ou $C_6$)-(alkyle en $C_1$ à $C_6$), sous réserve que l'un au moins de $R^2$, $R^3$ et $R^4$ soit impérativement un groupe A ; ou bien
(b) $R^2$ et $R^3$ forment, conjointement avec l'atome de carbone auquel ils sont attachés, un système cyclique ou bicyclique choisi dans le groupe comprenant un reste cycloalkyle en $C_3$ à $C_7$, cycloalcényle en $C_3$ à $C_7$, bicycloalkyle en $C_6$ à $C_{14}$, bicycloalcényle en $C_6$ à $C_{14}$ et des systèmes condensés à un noyau aryle et des systèmes condensés à un noyau hétéroaryle, contenant 8 à 15 atomes de carbone, l'un des noyaux de ces systèmes condensés à un noyau aryle et condensés à un noyau hétéroaryle étant aromatique et le noyau contenant le carbone auquel $R^2$ et $R^3$ sont attachés étant non aromatique, l'un des atomes de carbone du noyau aromatique étant facultativement remplacé par du soufre ou de l'oxygène, un ou plusieurs atomes de carbone du noyau non

aromatique étant facultativement remplacés par du soufre ou de l'oxygène et un ou plusieurs atomes de carbone du noyau aromatique étant facultativement remplacés par de l'azote, un ou deux atomes de carbone des groupes cycloalkyle ou bicycloalkyle étant facultativement remplacés par du soufre ou de l'oxygène, et le système cyclique ou bicyclique étant facultativement substitué avec un à cinq substituants choisis, indépendamment, dans le groupe consistant en un reste phényle, phényle substitué, alkyle en $C_1$ à $C_6$ et A, sous réserve qu'un et un seul de ces substituants soit un groupe A, et qu'un et un seul de ces substituants soit un groupe phényle ou phényle substitué, ce groupe phényle substitué portant ou plusieurs substituants choisis indépendamment dans le groupe des substituants alkyle en $C_1$ à $C_6$, alkylthio en $C_1$ à $C_6$, halogéno et trifluorométhyle ; et $R^4$ est de l'hydrogène, un groupe $XR^{10}$ ou A ;

A est un groupe hydrocarboné contenant 4 à 16 atomes de carbone et 0, 1 ou 2 doubles liaisons ;

X représente O, S, SO, $SO_2$, NH, $NR^{23}CO$ ou $NSO_2R^{24}$, où $R^{23}$ est de l'hydrogène ou un groupe alkyle en $C_1$ à $C_6$ et $R^{24}$ est un groupe alkyle en $C_1$ à $C_6$, phényle ou (alkyle en $C_1$ à $C_3$)phényle ;

$R^5$ est un groupe alkylthio en $C_1$ à $C_6$ qui peut être attaché à l'un ou l'autre des noyaux du système bicyclique de noyaux,

$R^{10}$ est choisi dans le groupe comprenant un reste cycloalkyle en $C_4$ à $C_{12}$, alkyle linéaire ou ramifié en $C_4$ à $C_{12}$, (cycloalkyle en $C_4$ à $C_{12}$)-(alkyle en $C_1$ à $C_6$), phényl-(alkyle en $C_1$ à $C_6$), (phényle substitué)-(alkyle en $C_1$ à $C_6$), (alkyle en $C_1$ à $C_6$)-phényle, (alkyle en $C_1$ à $C_6$)-(phényle substitué), des thiazoles substitués, des benzothiazoles substitués et des pyridines substituées ; les substituants du noyau phényle substitué, des thiazoles substitués, des benzothiazoles substitués et des pyridines substituées étant choisis dans le groupe des substituants alkoxy en $C_1$ à $C_4$, alkylthio en $C_1$ à $C_4$, alkyle en $C_1$ à $C_6$, halogéno et trifluorométhyle ;

B, D et E sont choisis entre de l'azote et du carbone, sous réserve qu'un ou deux de B, D et E représentent de l'azote,

ou d'un sel pharmaceutiquement acceptable de ce composé, comprenant :

la réaction d'un composé de formule

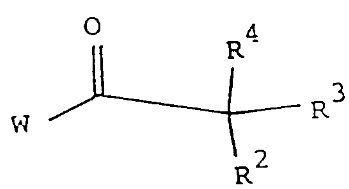

IV

dans laquelle W est un radical chloro ou bromo et $R^2$, $R^3$ et $R^4$ sont tels que définis ci-dessus, avec une amine de formule $R^1NH_2$, dans laquelle $R^1$ est tel que défini ci-dessus, et un accepteur d'acide.

**2.** Procédé suivant la revendication 1, dans lequel le composé de formule IV est obtenu par réaction d'un composé de formule

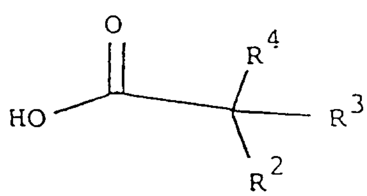

III

dans laquelle $R^2$, $R^3$ et $R^4$ sont tels que définis dans la revendication 1, avec un agent de chloration ou de bromation.

**3.** Procédé suivant les revendications 1 et 2, dans lequel l'accepteur d'acide est choisi dans le groupe comprenant la diméthylaminopyridine, la pyridine et la triéthylamine et l'agent de chloration ou de bromation est choisi dans le groupe comprenant le chlorure d'oxalyle, le bromure d'oxalyle, le chlorure de thionyle, le bromure de thionyle, le trichlorure de phosphore, le tribromure de phosphore, le pentachlorure de phosphore, le pentabromure de phosphore, l'oxychlorure de phosphore et l'oxybromu-

re de phosphore.